# EUROPEAN PATENT APPLICATION

(11) **EP 4 272 652 A1**
(43) Date of publication of application: **08.11.2023**
(21) Application number: 22171444.7
(22) Date of filing: 03.05.2022
(51) Int. Cl.: A61B 6/04, A61B 5/00, A61N 5/10, G01R 33/28

(54) **PATIENT/EQUIPMENT POSITIONING ASSISTANCE BY DEPTH IMAGING**

(71) Applicant: Koninklijke Philips N.V., 5656 AG Eindhoven (NL)
(72) Inventor: SENEGAS, Julien, Eindhoven (NL); KRUEGER, Sascha, Eindhoven (NL); WEISS, Steffen, Eindhoven (NL); MAZURKEWITZ, Peter Caesar, Eindhoven (NL); TANTTU, Jukka Ilmari, Eindhoven (NL); KRUISKAMP, Marinus Johan, Eindhoven (NL)
(74) Representative: Philips Intellectual Property & Standards

(57) **Abstract**

A device, system, method (100) and computer-program product are disclosed for assisting in the positioning of objects, including a subject and/or auxiliary equipment, on a subject support in an imaging and/or therapy session. The method comprises acquiring (101) image/spatial data that comprises depth/3D information, using a camera system, of the subject support having (an) object(s) placed thereon. The method comprises obtaining (111) reference data, comprising reference depth/3D information, representative of the subject support without the object(s) placed thereon. From the image and/or spatial data, at least one geometric attribute of the (or each) object is determined, in which this determining (104) comprises detecting (105) the object(s) by taking the reference depth/3D information data and the depth/3D information in said acquired image and/or spatial data into account.

## Description

### FIELD OF THE INVENTION

The present invention relates to the field of diagnostic imaging and therapy systems, and more specifically to a device, system, workstation, method and/or computer program product for monitoring and assisting in the positioning and/or orienting of a subject and/or auxiliary equipment in an imaging and/or therapy environment, e.g. to provide support to an operator in a preparation phase of an imaging and/or therapy session.

### BACKGROUND OF THE INVENTION

Preparing a patient for an examination that relies on diagnostic imaging technology (e.g. to diagnose a medical condition and/or plan a treatment) and/or for a treatment session is typically a time-consuming task that heavily relies on manual interventions by trained personnel. Typically, a skilled operator needs to carefully perform the preparation and setup for a session in accordance with an established protocol and/or specific guidelines regarding the positioning of the patient and the use of auxiliary devices.

For example, the operator may need to place positioning devices, such as knee supports, pads, cushions or head rests, to achieve a stable resting position of the patient throughout the imaging or treatment session. For the sake of consistency, this position may need to conform as closely as possible to a reference position, or, more generally, to a reference spatial configuration of the body, which may depend on the specific type of examination or treatment that is intended or may even be specific to the particular patient, symptoms and/or conditions of the patient and/or other unique factors. It is also often essential to reproduce the exact patient setup over consecutive sessions for the same patient, e.g. in different imaging sessions and/or in related therapy sessions.

For example, it may be strongly preferable to acquire (diagnostic) images of the same patient using different modalities and/or that are representative of different points in time in a way such that the images are as similar to each other as possible, e.g. such that the image content is easily comparable or can be easily processed by dedicated software. Likewise, it may be important that the acquired images depict the spatial configuration of the body (or a relevant part thereof) as closely as possible to the state in which it will be reproduced in (a) future therapy session(s), e.g. such that a radiotherapy (or different type of treatment) can be accurately and reliably planned and executed. In radiation therapy, for example, it is common to require that the exact same patient setup is reproduced as closely as possible for many, e.g. even up to forty, separate radiation delivery sessions.

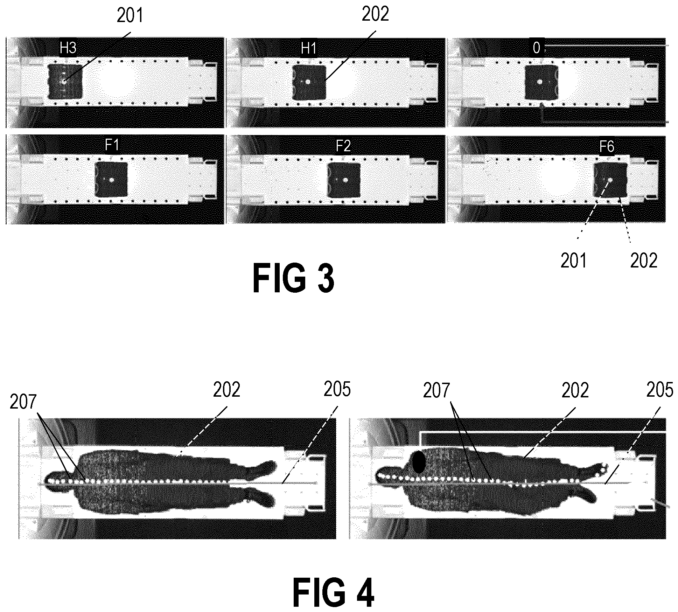

To allow an accurate positioning of auxiliary equipment, e.g. positioning devices such as head and/or knee supports, in a relatively fast and convenient manner, patient supports (i.e. the patient table or examination/treatment couch) may be equipped with mechanical means to provide a fixed, discrete set of positions where such auxiliary devices can be mounted, e.g. by bolts, screws, pegs, clamps or the like. These positions may be easily recorded and referenced, e.g. by labeling these discrete positions and referencing the position(s) to use in a patient file, guidelines and/or other such information repositories. As an example, 12 equidistantly spaced linear positions along the longitudinal axis of the support may be labeled from H4 to F7 ("head 4", "head 3", ..., 0, "feet 1", ..., "feet 7"). An example of such discrete indexing is e.g. shown in Fig. 3. A device, such as a knee support, may thus be positioned at these specific discrete positions. The position that is used, or is to be used, can be conveniently marked in an annotation in the patient records and/or in procedural guidelines.

Diligent quality assurance procedures may typically require the reporting of all relevant setup information in detail for each session, i.e. to be able to compare, evaluate and/or reproduce the setup. It will be understood that a manual workflow for positioning the patient and auxiliary positioning devices, as well as the recording of the relevant information, may be a time-consuming and error-prone task.

It is known in the art, e.g. specifically in radiotherapy planning, to use radio frequency identification tags (RFIDs) to check the presence of the correct type of positioning devices and their respective locations. For example, such system may use a plurality of RFID sensors and tags, which can be integrated in a set of positioning devices and indexing and/or holder devices. This allows the system to use a set of registered devices, i.e. which are accordingly equipped with the system's components.

While RFIDs can help to automate, reproduce and safeguard the positioning setup, reporting and reproduction workflow, this has also some limitations. For example, a typical patient setup may be only recorded to some extent, i.e. some of the complexity of the configuration may be lost or might still need to be recorded manually. Such system is typically also not able to deal with custom or third-party devices when these are not equipped with the required RFID components, while adding the required RFID components to the custom or third-party device may also be relatively difficult for various technical, but potentially also other, reasons (e.g. compliance, quality assurance, maintenance, manufacturer support, calibration and/or certification).

Since an RFID-equipped system typically relies on many co-operating components, e.g. RFID tags and sensors, the likeliness of an error generally increases in line with the number of devices over which the components are distributed, i.e. since each tag and sensor may fail independently. Another potential disadvantage is the limited range of RFID signals, being essentially a technique intended for short range communication, such that the position detection might fail for longer distances between the tag and sensor.

It is also to be noted that some positioning aid devices are not suitable for installation along a fixed predefined linear and/or raster index, or the available (discrete) positions may be too limited for a particular case. The proper use of a device may also imply other parameters that are not easily encoded in a single position parameter.

Examples of devices that are not easily positioned in a reproducible manner by relying on solely a discrete raster and/or linear coordinate system may include support wedges, flexible devices, padding, cushions and custom-made patient-specific positioning devices. For example, when a prior-art RFID-based system is used, such devices will typically not be included in the setup report automatically, which raises a potential risk due to errors in (manual) reporting, may decrease time-efficiency due to additional manual reporting steps, and/or limits the ability to reproduce the configuration accurately.

More generally, complex patient setups may be difficult to reproduce, e.g. in cases where detailed step-by-step guidance to achieve the correct result needs to be followed, when using a prior-art RFID-based system or even using any manual or (partially) automated approach that uses a discrete positioning grid.

Examinations to plan and/or monitor a treatment may involve magnetic resonance imaging, such that a system for assisting in the positioning of the patient and auxiliary devices would preferably be suitable for use in such MRI sessions (preferably, in addition to being usable in related therapy and/or imaging sessions using other imaging modalities). This illustrates another possible disadvantage of RFID-based systems. Such system relies on radiofrequency signal transmission, which can be disrupted by the magnetic fields and radiofrequency signals used by the MRI system. This may also imply an RF safety concern, since the RFID technology could interfere with MR imaging and/or other medical devices, i.e. putting the patient and/or equipment at risk. While additional safety measures and design choices might address this problem, this would inevitably lead to increased costs and efforts in the integration and implementation thereof.

It is known in the art to position a movable patient table relative to an imaging system based on camera observation. For example, US 10181074 B2 describes such approach, in which a frozen camera image is used, on which an operator can define reference location information. Then, the examination table can be moved to bring the reference information into congruence with an acquisition region of the system. It is also known, see e.g. US 2009/182221 AI, to store camera image positions of the patient and of an MRI reception coil in a medical picture archiving and communication system (PACS) server, such that this information can be presented to an operator in a subsequent session to reproduce the setup. While camera-based patient positioning may offer many advantages, a need remains in the art for an approach to provide easy, strongly automated, reliable, reproducible, versatile and accurate positioning of the patient and/or any auxiliary equipment, e.g. positioning aids, and accurate and/or reliable recording of such position information. Preferably, a positioning system would be MRI-compatible and able to handle arbitrary equipment items, e.g. requiring little or no modification of positioning devices, even when supplied by a different manufacturer or patient-specific, e.g. 3D printed for a particular patient.

### SUMMARY OF THE INVENTION

It is an object of embodiments of the present invention to provide in good and/or efficient means and methods to assist in the positioning and/or orienting of a subject (e.g. patient) and/or equipment in a diagnostic imaging and/or treatment system (and/or to facilitate the recordation of such position information).

The equipment, referred to hereinabove, may generally relate to one or more items that can be placed in the system (e.g. thus in the imaging and/or treatment environment) to support, stabilize, orient, shape (e.g. deform, flex) or otherwise affect the spatial configuration of the patient, i.e. may refer to positioning devices such as pads, supports, cushions, masks, stereotactic reference devices, physical markers, and the like. Optionally, the equipment may also comprise (or even consist of) other devices that are freely, or at least variably, placeable, orientable, shapeable, ... in relation to the subject, e.g. sensor devices, cardiogram monitors, breathing belt equipment, etc., e.g. which have a function other than, or not limited to, being merely a positioning aid. The latter 'other' devices may have a position and/or spatial relation to the subject, in use thereof, which could be relevant to record and/or reproduce accurately and/or to configure in accordance with a predetermined specification.

The equipment may exclude fixed components of the imaging system, i.e. that are essentially static in the environment or fixed to a movable (typically actuated and controlled) part of the imaging/treatment system, e.g. to a gantry, a magnet bore enclosure, a linear accelerator, a robotic arm or another key component of the imaging/treatment system as such. Nonetheless, embodiments are not necessarily limited by this illustrative exclusion, e.g. in some applications it may even be advantageous to assist in the positioning of such (e.g. essential) system components, e.g. a radiation source, an imaging detector, .... For example, this may be the case in specific applications where such key component of an imaging/therapy system substantially relies on manual positioning, e.g. a portable (non-mechanized) C-arm imaging system, a bed-side projection radiography system, an isolated gamma radiation source, etc.

It is an advantage of embodiments of the present invention that a patient and positioning devices can be placed in an imaging and/or therapy environment by a partially automated, machine-assisted, procedure.

It is an advantage of embodiments of the present invention that the position and/or spatial configuration (e.g. orientation, shape, relative position/orientation, ...) of a patient and/or positioning aids can be easily and accurately recorded, e.g. such that the same configuration can be easily, accurately and/or reliably reproduced.

It is an advantage of embodiments of the present invention that the burden of time-consuming tasks in setting up a patient for an imaging or treatment procedure can be reduced, e.g. such that an operator can accordingly work more efficiently. For example, disadvantages of a substantially (entirely or predominantly) manual workflow can be reduced, such as being error-prone, carrying a time expenditure cost and/or requiring the operator to perform many quality checks entirely manually.

It is an advantage of embodiments that a good reproducibility and/or consistency in positioning the patient (and/or equipment) can contribute to an efficient and/or accurate diagnosis and/or treatment.

It is an advantage of embodiments of the present invention that manual interventions required in positioning a patient and/or equipment can be reduced and/or simplified.

It is an advantage of embodiments of the present invention that training requirements and/or experience of staff required for properly positioning a patient (including the use of positioning aids) can be reduced or relaxed. It is also an advantage of embodiments of the present invention that errors and mistakes in positioning of the patient and positioning devices can be reduced or avoided.

It is an advantage of embodiments of the present invention that embodiments of the present invention may provide a real-time, e.g. substantially continuously updated, position tracking, positioning guidance and/or position recording (and/or tracking, guidance and/or recording of other geometric parameters).

It is an advantage of embodiments of the present invention that assistance can be provided in positioning auxiliary positioning aids and recording the position (and possibly other spatial configuration properties) thereof, without unduly limiting the possible positions and/or configuration options, e.g. to a fixed, discrete set of selectable positions.

It is an advantage of embodiments of the present invention that mechanical means for attaching and/or fixing a positioning device to the patient support table are not required (if not deemed necessary or advantageous for medical and/or other practical reasons) to use a method and device in accordance with embodiments.

It is an advantage of embodiments of the present invention that the position and/or other spatial configuration parameters of the patient and of positioning devices can be easily recorded for later use and retrieved for reuse when previously stored. Not only a position with respect to the patient table, but also other geometric properties, such as orientation, shape, elevation and/or deformation, of the patient and/or of positioning devices can be recorded.

It is an advantage of embodiments of the present invention that the positioning guidance provided by embodiments can be applied, without modification, registration, mathematical modeling and/or other initial configuration cost, to generic, non-standard, third-party, custom-made and/or, generally, arbitrary auxiliary devices. For example, any positioning device may be used in combination with embodiments of the present invention, in so far that it can be observed by its camera system and detected automatically. A learning phase or extensive setup or configuration to recognize a specific device by the system in accordance with embodiments is not required, e.g. it is not necessary to train or configure the system for a specific shape or appearance of the positioning device. Hence, new devices that were not used before in combination with the system, e.g. that were not previously observed by the camera, can be readily used. A specific hardware modification of the auxiliary device is thus also not required.

It is an advantage of embodiments of the present invention that the positioning guidance provided is not limited to a short range of wireless communication between sensors and tags.

It is an advantage of embodiments of the present invention that embodiments of the present invention are compatible with, usable in and/or easily integrated in magnetic resonance imaging systems, computed tomography imaging systems and/or radiotherapy systems, e.g. without substantial interference and/or other safety risks. For example, in MRI, camera observation may be used from outside of (e.g. at a sufficient distance from) a volume of space where a camera might interfere with the MRI imaging. For example, a camera or combination of cameras may be positioned outside or near an edge of the primary magnet enclosure, e.g. close to or on a flange of the bore, from where it may observe the patient and any equipment of interest without interfering with the sensitive radio-frequency/magnetic gradient systems.

It is an advantage of embodiments of the present invention that positioning guidance, recording, quality assurance and/or management can be provided in combination with potentially any diagnostic imaging system, such as computed tomography (CT), magnetic resonance imaging (MRI), projection radiography, single photon emission tomography (SPECT), positron emission tomography (PET) and/or other imaging modalities, and/or in combination with any treatment modality in which accurate positioning of the patient (and/or equipment items) is important, e.g. a radiotherapy system, such as a linear accelerator (LINAC) based treatment system, a robotic and/or image-guided surgery suite, and potentially other therapy systems. This may also include combined imaging and therapy systems, e.g. in which a CT and/or MRI system (without limitation to said combination) is integrated in the treatment system, e.g. an MR-LINAC system.

It is an advantage of embodiments of the present invention that a good accuracy, efficiency and/or effectiveness can be achieved in radiotherapy, and/or, generally, in image-guided therapies, e.g. HIFU. For example, a high-quality image-guided treatment, e.g. MRI or CT guided radiotherapy, can be achieved. It is an advantage of embodiments of the present invention that MRI and/or other types of imaging can be achieved with good image quality and reproducible representation of the targeted anatomy. It is an advantage of embodiments of the present invention that short session times can be achieved in MRI, CT and/or other imaging modalities, e.g. due to reduced time requirements for initial setup of the patient.

It is also an advantage that camera observation is commonly used in MRI imaging to monitor the patient. Therefore, an existing camera system may be easily upgraded/upgradable in order to implement an embodiment of the present invention, and/or suitable MRI-compatible camera systems may be readily available for use in embodiments of the present invention. Camera observation may be commonly used in diagnostic imaging and/or treatment environments, e.g. in CT, PET, SPECT, radiotherapy, ... It is an advantage of embodiments of the present invention that a pre-installed camera system of such imaging or therapy suite may be easily adapted for use with the present invention, e.g. to provide positioning guidance and/or recording in addition to existing patient observation functionality. For example, existing camera devices may be replaced, extended and/or upgraded, e.g. to support depth imaging where it was not previously available in combination with suitable processing as explained further hereinbelow.

It is an advantage of embodiments of the present invention that different diagnostic images (2D, 3D or even timeseries or sets of images), e.g. acquired in different examinations for a same patient on the same or heterogenous imaging modalities, can be accurately, efficiently and/or easily registered to each other, e.g. due to a good consistency and reproducibility of the patient position, orientation and/or spatial configuration between the different sessions.

It is an advantage of embodiments of the present invention that image registration errors and/or artefacts in registered images can be reduced, and therefore that propagation of such errors/artefacts to further images, treatment plans, treatments and/or other kinds of information or end-results derived therefrom or reliant thereon may also be reduced.

It is an advantage of embodiments of the present invention that the use of depth imaging allows to gather information about not only an in-plane position (and/or orientation, ...) of equipment (and of the patient) with respect to the (typically horizontal) patient table, but also height (orientation, shape, ...) information in the normal direction to the plane, even though the latter may also be relevant for accurate and reproducible positioning. For example, this allows an elevation level and/or angle to be determined, recorded and/or accurately reproduced, e.g. of a leg, a foot, an arm, a hand, the trunk (or a specific part thereof) and/or the head, e.g. when such body part(s) is (are) supported by (a) positioning device(s).

Depth imaging may also, advantageously, avoid false detection due to shadows, inhomogeneous lighting and/or irrelevant, (substantially) thin objects on the patient table, e.g. a sheet of paper. Conventional two-dimensional photographs (or video), such as can be obtained by a monochrome or color camera, would typically require more complex image processing and/or would struggle with distinguishing such nuisance objects and/or lighting artefacts from the object(s) of interest.

This also implies that, in embodiments of the invention, e.g. due to (or at to some extent due to) the use of depth imaging technology, the implicit bulk nature of a device (equipment item) can be relied upon to detect the object, and potentially also algorithmically separate different objects, e.g. without a need to train or configure the system for a specific shape or other properties of the object. For example, this bulk nature may be implicit for a device that is instrumental in positioning and/or immobilizing the patient, e.g. which may typically involve at least some substantial physical volume and/or mass to achieve the intended purpose (in a robust and stable manner).

A method, computer program product, device, system and/or workstation in accordance with embodiments of the present invention achieves the above objective.

In a first aspect, the present invention relates to a method for assisting in the positioning and/or orienting of objects, e.g. a subject and/or at least one auxiliary equipment item, on a subject support (e.g. a patient couch, imaging/therapy table, ...) in an imaging and/or therapy session (i.e. using an imaging and/or therapy system). The method comprises acquiring image and/or spatial data, using a camera system, of the subject support having at least one of said objects placed thereon, wherein said image and/or spatial data comprises depth (and/or 3D) information. The method further comprises obtaining reference data representative of the subject support without said at least one object placed thereon, the reference data comprising reference depth (and/or 3D) information.

The method comprises determining, from the image and/or spatial data, at least one geometric attribute of the (or each of said) at least one object, in which this determining of the at least one geometric attribute comprises detecting the at least one object by at least taking the reference depth (and/or 3D) information in the reference data and the depth (and/or 3D) information in the acquired image and/or spatial data into account.

Thus, the method may comprise detecting positions, orientations and/or other spatial properties of the subject (e.g. a patient) and/or the auxiliary equipment item(s) and/or detecting spatial relationships between the subject and the equipment and/or between different equipment items. This spatial configuration information may be used to assist in the patient and/or equipment positioning in a preparation step of the imaging and/or therapy session, and/or may be stored for future reference.

In a method in accordance with embodiments of the present invention, the step of detecting may comprise determining three-dimensional points for which the corresponding acquired depth (and/or 3D) information differs substantially from the corresponding reference depth (and/or 3D) information, and associating said points, and/or (e.g. automatically determined) clusters of said points, with the object or objects being detected.

In a method in accordance with embodiments of the present invention, the reference data may comprise a three-dimensional geometric model of the subject support and/or at least one depth image of the subject support.

For example, the reference data may comprise a three-dimensional model of the subject support, e.g. a Computer Aided Design (CAD) model. Such model may thus, for example, comprise a 3D geometric model of the scanner couch, and optionally also other relevant, e.g. visible (to the camera system), components of the system.

In a method in accordance with embodiments of the present invention, obtaining the reference data may comprise receiving a current position of the subject support from an automated subject support actuation system and/or determining said current position from said acquired image and/or spatial data. Obtaining the reference data may further comprise determining the reference depth (and/or 3D) information based on the current position in combination with a three-dimensional geometric model of the subject support (e.g. defined as function of its position) and/or based on the current position and a set of reference depth (and/or 3D) images of the subject support (e.g. corresponding to a plurality of different positions).

In a method in accordance with embodiments of the present invention, the steps of acquiring the image and/or spatial data and detecting the at least one of the objects may be repeated. After each iteration (of said repetition), the reference data may be updated to include the detected object or objects, such that the updated reference data represents the subject support with thereon the object or objects detected up to said iteration. Then, in a next iteration, one or more further objects may be placed on the subject support to be detected, e.g. to guide an operator in placement of the further object(s) while the previously detected object(s) remain substantially static.

In a method in accordance with embodiments of the present invention, the at least one geometric attribute may comprise a discrete subject support index, selected from a predetermined discrete ordinal set of subject support indices (e.g. discrete reference longitudinal table positions as used for reference in radiotherapy setups), to identify a position of the detected object associated with the at least one geometric attribute along a (principal, e.g. typically a longitudinal) direction of the subject support.

In a method in accordance with embodiments of the present invention, the at least one geometric attribute may comprise at least one value indicative of the position and/or of the orientation of the object associated therewith, e.g. in one predetermined direction (e.g. a longitudinal table coordinate), two predetermined directions (e.g. longitudinal and lateral coordinates) and/or three predetermined directions (e.g. further including a height, e.g. vertical, coordinate), and/or the equivalents thereof with respect to orientation (e.g. rotation or rotations around one, two or three axes).

In a method in accordance with embodiments of the present invention, the at least one geometric attribute may comprise a center line of the or each detected object, e.g. along a predetermined axis (e.g. a longitudinal axis of the subject support) or with respect to a straight line detected as a principal axis of the object, e.g. such as to show deformation of the object with respect to a predetermined, mean, best-fit or similarly representative straight axis, as automatically determined, of the object.

In a method in accordance with embodiments of the present invention, the at least one geometric attribute may comprise a straight, segmented or curved line, or e.g. a set of points indicative of such line, that is representative of the center line of the detected object.

A method in accordance with embodiments of the present invention may comprise presenting the determined at least one geometric attribute of the detected object or objects (e.g. for each object) to an operator. It will be understood that some differentiation may be applied, e.g. determining and/or showing (a) different attribute(s) for the patient and for an inanimate object. It will also be understood that for the purposes of presentation (a) different attribute(s) may be determined and presented than for the purposes of information recordation (data storage for later use). This does not preclude the same set of attributes being used for the patient and for an inanimate object, or for presentation and for storage, nor does it exclude potential overlap between such sets for these different types of object (patient vs. equipment) or different intended use (presentation vs. recordation).

In a method in accordance with embodiments of the present invention, this presenting may comprise presenting the at least one geometric attribute in a visual form to the operator in an overlay on at least one camera image as acquired, and/or on a geometric model abstracted from the acquired image and/or spatial data and/or from the obtained reference data.

In a method in accordance with embodiments of the present invention, the shape, area (e.g. the projected surface on the image plane) and/or volume of the detected object or objects may be presented to the operator as a (e.g. color) overlay or contour on said at least one camera image and/or model of the scene.

In a method in accordance with embodiments of the present invention, the center-line of the detected object or objects may be presented to the operator on said at least one camera image and/or model of the scene. Additionally, the center-line may be presented in 3D, e.g. in a 3D model, on two (e.g. orthogonal) views, by showing a height (vertical) profile in addition to a (horizontal) plane presentation or by another visualization that allows the operator to assess both in-plane alignment and normal-to-plane alignment simultaneously.

A method in accordance with embodiments of the present invention may comprise repeatedly performing the image and/or spatial data acquisition, determining the at least one geometric attribute, and presenting the at least one geometric attribute (e.g. repeating that order of the steps), so as to dynamically guide the operator in positioning and/or orienting the detected object and/or objects.

A method in accordance with embodiments of the present invention may comprise storing the geometric attribute(s) in a data storage for future reference in another session, for quality assurance, for workflow documentation, and/or for use in further processing and/or evaluation of information gathered in the image and/or therapy session.

A method in accordance with embodiments of the present invention may comprise retrieving the geometric attribute(s) stored in a previous session, and including the thus retrieved information (not necessarily excluding any intermediate further processing, transformation or other type of digital manipulation of the retrieved information) in said presentation for comparative purposes.

In a method in accordance with embodiments of the present invention, the image and/or spatial data may be acquired from one or more camera positions arranged above the subject support such as to obtain a two-dimensional and/or three-dimensional overhead view of the subject support.

In a method in accordance with embodiments of the present invention, acquiring the image and/or spatial data may further comprise acquiring at least one two-dimensional image from an optical camera, comprised in the camera system, wherein said optical camera is sensitive to one or more spectral bands in the infrared and/or human visible spectrum. The at least one two-dimensional image may comprise a monochrome image, a color image and/or a multispectral image.

In a method in accordance with embodiments of the present invention, acquiring the image and/or spatial data may comprise processing a plurality of two-dimensional images that are acquired substantially simultaneously by a stereo or multi camera setup from a plurality of different vantage points so as to determine said depth (and/or 3D) information (e.g. using an algorithm known in the art to infer depth (and/or 3D) information from a plurality of concomitantly acquired images from different viewing perspectives).

In a method in accordance with embodiments of the present invention, said imaging and/or therapy session may comprise a magnetic resonance imaging session, a computed tomography imaging session, an imaging session using another diagnostic imaging modality (e.g. projection radiography, mammography, echography, ...), a nuclear medicine imaging session (e.g. SPECT, PET, ...), a radiotherapy session and/or a different (typically automated or machine-assisted) therapeutical session.

In a second aspect, the present invention relates to a device for assisting in the positioning and/or orienting of a plurality of objects, including a subject and/or at least one auxiliary equipment item, on a subject support in an imaging and/or therapy session, the device comprises a camera system for acquiring image and/or spatial data of the subject support, while having at least one of said objects placed on said subject support, in which the camera system is adapted for acquiring depth (and/or 3D) information. The device further comprises a processor and an output.

The processor is adapted for obtaining reference data representative of the subject support without the at least one object placed thereon, in which the reference data comprises reference depth (and/or 3D) information. The processor is also adapted for: detecting the at least one object by at least taking the reference depth (and/or 3D) information in the reference data and the depth (and/or 3D) information in the acquired image and/or spatial data into account, for determining at least one geometric attribute of the (or each of) the detected object(s) placed on the subject support, and for outputting, via the output, the at least one geometric attribute.

In a device in accordance with embodiments of the present invention, the processor may be adapted for detecting the at least one object by determining three-dimensional points for which the corresponding acquired depth (and/or 3D) information differs substantially from the corresponding reference depth (and/or 3D) information.

In a device in accordance with embodiments of the present invention, the processor may be adapted to associate said points, and/or determined clusters of said points, with the at least one object (e.g. associating different clusters with different objects being detected).

In a device in accordance with embodiments of the present invention, the processor may be adapted to repeat the steps of acquiring the image and/or spatial data, via the camera system, and detecting at least one of the objects, in which, after each iteration, the reference data is updated to include the detected object or objects, such that the updated reference data represents the subject support with thereon the object or objects detected up to said iteration, and such that, in a next iteration, one or more further objects can be placed on the subject support to be detected.

In a device in accordance with embodiments of the present invention, the processor may be adapted to determine said at least one geometric attribute, in which the at least one geometric attribute may comprise a discrete subject support index, selected from a predetermined discrete ordinal set of subject support indices, to identify a position of the detected object associated with said at least one geometric attribute along a longitudinal direction of said subject support.

In a device in accordance with embodiments of the present invention, the processor may be adapted to determine said at least one geometric attribute, in which the at least one geometric attribute may comprise at least one value indicative of the position and/or of the orientation of the detected object associated therewith.

In a device in accordance with embodiments of the present invention, the processor may be adapted to determine said at least one geometric attribute, in which the at least one geometric attribute may comprise a centerline of the detected object associated therewith.

In a device in accordance with embodiments of the present invention, the camera system may comprise at least one camera arranged above the subject support such as to obtain a two-dimensional and/or three-dimensional overhead view of the subject support.

In a device in accordance with embodiments of the present invention, the camera system may comprise a depth camera (e.g. a device suitable for determining 3D information of the observed scene).

In a device in accordance with embodiments of the present invention, the camera system may comprise at least one optical camera for acquiring a two-dimensional image, wherein said optical camera is sensitive to one or more spectral bands in the infrared and/or human visible spectrum, e.g. adapted to acquire a monochrome image, a color image and/or a multispectral image. It is an advantage that the geometric attribute(s) can be visually presented on the optical camera image, which is easily interpretable by the operator, e.g. akin to normal human vision.

In a device in accordance with embodiments of the present invention, the camera system may comprise a plurality of two-dimensional optical cameras to acquire substantially simultaneously two-dimensional images from a plurality of different vantage points, wherein the camera system is adapted determine said depth (and/or 3D) information from said two-dimensional images, e.g. using stereo or multi-camera image processing.

In a device in accordance with embodiments of the present invention, the reference data obtained by the processor may comprise a three-dimensional geometric model of the subject support and/or at least one depth image of the subject support.

A device in accordance with embodiments of the present invention may comprise an input for receiving a current position of the subject support from an automated subject support actuation system and/or the processor may be adapted for determining said current position from said acquired image and/or spatial data. The processor may be furthermore adapted to determine said reference depth (and/or 3D) information based on said current position and a three-dimensional geometric model of the subject support defined as function of its position and/or based on said current position and a set of reference depth (and/or 3D) images of the subject support corresponding to a plurality of different positions.

In a device in accordance with embodiments of the present invention, the output may comprise a display monitor, wherein said processor may be adapted for presenting, via said display monitor, a visual representation of the determined at least one geometric attribute for the detected object or objects to an operator.

In a device in accordance with embodiments of the present invention, the processor may be adapted for repeatedly performing the image and/or spatial data acquisition, determining the at least one geometric attribute, and presenting the at least one geometric attribute, so as to dynamically guide the operator in positioning and/or orienting the detected object and/or objects.

In a device in accordance with embodiments of the present invention, the processor may be adapted for presenting said visual representation in an overlay on at least one camera image as acquired by the camera system and/or on a geometric model abstracted from the acquired image and/or spatial data and/or from the obtained reference data.

In a device in accordance with embodiments of the present invention, the processor may be adapted to visually present the shape, area and/or volume of the detected object or objects. In a device in accordance with embodiments of the present invention, the geometric attribute(s), e.g. a center point, orientation vector, box contour (or another simple geometric 2D or 3D shape that may be fitted to the object) and/or principal axes of the (or each) object, may be shown in the visual presentation.

In a device in accordance with embodiments of the present invention, the centerline may comprise, and/or may be visually presented via the display monitor as, a straight, segmented or curved line, or a set of points indicative of such line, in which the line is representative of a centerline of the detected object.

In a device in accordance with embodiments of the present invention, the geometric attribute(s) may be shown, in the visual presentation, in 2D or 3D. For example, the centerline may be indicated on at least two (e.g. orthogonal) views, or a height (e.g. vertical) plot of the centerline may be shown in addition to a planar (e.g. horizontal) view, e.g. an accordingly annotated overhead camera image.

A device in accordance with embodiments of the present invention may comprise a data storage interface (and/or may comprise such data storage), in which the processor may be adapted for storing the at least one geometric attribute in a data storage, via the data storage interface, for future reference.

In a device in accordance with embodiments of the present invention, the processor may be adapted to retrieve the at least one geometric attribute stored in a previous session, and to include the retrieved information (potentially after further processing of the information) in said visual representation (provided via the display) for comparative purposes.

In a third aspect, the present invention relates a medical imaging system, e.g. a magnetic resonance imaging system or a computed tomography imaging system, or a (e.g. radio-) therapy system, wherein said imaging and/or therapy system is adapted to perform a method in accordance with embodiments of the first aspect of the present invention and/or comprises a device in accordance with embodiments of the second aspect of the present invention.

In a fourth aspect, the present invention relates a workstation for a medical imaging system or (e.g. radio-) therapy system, wherein the workstation is adapted to perform a method in accordance with embodiments of the present invention and/or comprises a device in accordance with embodiments of the present invention.

In a fifth aspect, the present invention relates to a computer-program product for performing, when executed on a computer, a method in accordance with the first aspect of the present invention.

The independent and dependent claims describe specific and preferred features of the invention. Features of the dependent claims can be combined with features of the independent claims and with features of other dependent claims as deemed appropriate, and not necessarily only as explicitly stated in the claims.

### BRIEF DESCRIPTION OF THE DRAWINGS

Fig. 1 shows a method in accordance with embodiments of the present invention.
Fig. 2 shows a device in accordance with embodiments of the present invention.
Fig. 3 illustrates an example of detection of a knee positioning device and its position index along the patient table direction, for different positions thereof, using embodiments in accordance with the present invention.
Fig. 4 illustrates an example of detection and visualization of a patient's body and the body's centerline along the table direction, for two different spatial configurations of the body, using embodiments in accordance with the present invention.

The drawings are schematic and not limiting. Elements in the drawings are not necessarily represented on scale. The present invention is not necessarily limited to the specific embodiments of the present invention as shown in the drawings.

### DETAILED DESCRIPTION OF EMBODIMENTS

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

In a first aspect, the present invention relates to a method for assisting in the positioning of a subject and/or auxiliary equipment in an imaging and/or therapy session, e.g. a magnetic resonance imaging or computed tomography imaging session (or using another imaging modality) and/or a radiotherapy session (or other treatment in which accurate positioning is deemed important). This assistance may include detecting positions, orientations and/or other spatial properties of the patient and/or the auxiliary equipment, and/or detecting spatial relationships between the subject and the equipment and/or between different equipment items. This information may be gathered for presentation in a convenient form, to record this information (e.g. for future reference in another session and/or as quality assurance or generic annotation), for comparing the information to references thereof (stored in an earlier session, e.g. as referred to hereinabove, and/or from a procedure description and/or obtained from any other suitable data source, e.g. a prescription, guidelines, ...), for confirming a correspondence between a present state and the relevant reference, for guiding an operator towards such congruence, and/or for other related functions.

Particularly, the method may be a computer-implemented method, i.e. an automated or semi-automated method that can be performed by dedicated processing hardware (e.g. using an application specific integrated circuit or circuits) and/or configured/programmed processing hardware, such as a computer or other general-purpose processor that is programmed for the specific task of performing the method and/or a configurable hardware platform (e.g. a field programmable gate array) configured for the specific task of performing the method. Combinations of application-specific hardware (e.g. ASIC) and/or configured hardware (e.g. FPGA) and/or one or more programmed processing devices (e.g. using a CPU, GPU or other suitable processor, possibly in combination with supporting hardware, e.g. as commonly found in computers) are also possible.

"Automated" and "semi-automated" may refer to a computer-implemented method implemented by a (e.g. digital) processor, controller and/or other such hardware, in which the method is performed in an autonomous or supervised autonomous mode, e.g. requiring only limited input from and/or interaction with an operator, e.g. to select or enter relevant parameters and/or configuration options, to start, stop and/or interrupt the procedure, to supervise the procedure and/or other such limited interactions.

Where reference is made to "camera," "depth camera," or similar components, it will be understood that such device, to which is referred, is not to be confused with the diagnostic imaging system as such (nor with a therapy system as such). Even though the "camera" may be implemented as a part of the imaging system, by default or by suitable modification, the primary purpose of the diagnostic imaging system will be understood to relate typically to an image acquisition process by different means, e.g. by ionizing radiation, radiofrequency signals, ..., and is not essentially (or at least not solely) based on the "camera" observation as such.

For example, even though the radiation detection component (i.e. image detector) of a PET or SPECT system can be referred to as a PET or SPECT "camera," it will be understood that this differs from the "camera" in the sense of the present disclosure. Cameras in the sense of the present disclosure may for example include conventional optical imaging cameras and depth imaging cameras. The former (conventional cameras) may generally relate to imaging cameras that detect (i.e. particularly in order to determine an image from) light in the (human) visible spectrum and/or infrared spectrum. The conventional camera may for example be adapted for monochrome, color and/or multispectral imaging. The latter (depth cameras) generally relates to technologies to detect depth information in addition to in-plane information (e.g. the two-dimensional projection plane of an image detector), e.g. using stereo imaging (or, more generally, camera imaging from multiple different viewpoints simultaneously), range cameras, LIDAR, RADAR, and/or other such techniques to determine points and/or surfaces in three-dimensional space, e.g. such that a surface contour (e.g. facing the camera) of (an) object(s) can be qualified at least to some extent in three dimensions. In this context, a depth camera is understood to refer generally to any suitable means for gathering spatial data characterizing the observed scene in three spatial dimensions, e.g. such that the (3D) position in space of points on surfaces (at least when not obscured from the camera viewpoint) of objects in the observed scene are determined.

The method in accordance with embodiments of the present invention may be used to assist in patient and/or equipment positioning in an imaging and/or therapy session, e.g. in a preparation step of a magnetic resonance imaging, computed tomography imaging and/or radiotherapy session, without limitation thereto. The method may also find application is a system or environment in which an imaging modality and a treatment modality are combined, e.g. radiotherapy delivery while concomitantly imaging the patient (e.g. by CT, MRI, ...). It will be understood that a magnetic resonance imaging (MRI) session refers to an examination using an MR imaging scanner, e.g. for diagnostic and/or medical purposes, such as to diagnose a condition of the subject, to plan a treatment, to monitor and/or control a treatment that is performed concomitantly, and/or other related, e.g. medical purposes. Likewise, a computed tomography imaging session refers to an examination using a computed tomography (CT) scanner.

Where reference is made to an imaging (e.g. MRI) session, it will be understood that this may refer to a procedure in which the (e.g. human) subject is positioned in an (e.g. MR) imaging system to perform the imaging procedure, after which one or a plurality of diagnostic images may be acquired (e.g. one or more MRI pulse and magnetic gradient sequences may be applied to acquire MRI data).

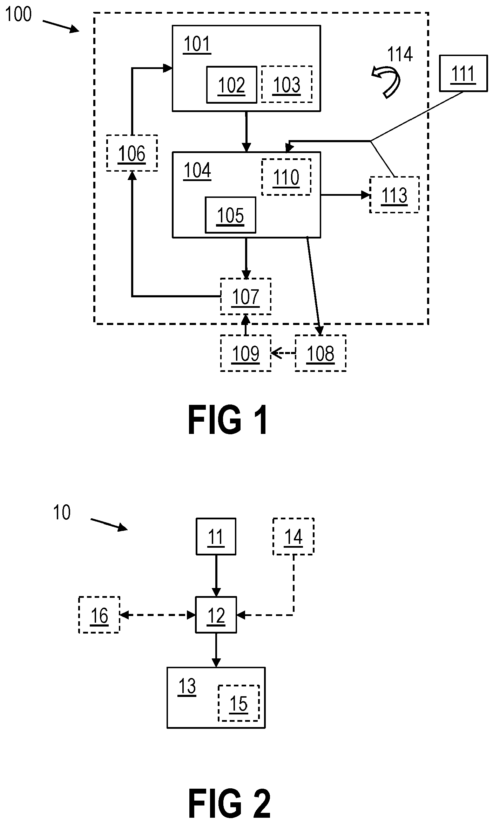

Referring to Fig. 1, an illustrative method 100 in accordance with embodiments of the present invention is shown. The method, e.g. computer-implemented method, 100 can be used to assist in the positioning of a subject and/or auxiliary equipment on a patient support in an imaging and/or therapy session, e.g. particularly in a preparation step of the session. Thus, the method is adapted for assisting in the positioning and/or orienting of a plurality of objects, in which these objects may particularly include a subject (e.g. patient) to which the session is to be applied (e.g. a subject to be diagnostically imaged and/or treated) and/or at least one auxiliary equipment item, such as a positioning aid.

The method 100 comprises acquiring 101 image and/or spatial data of the patient support, having one or more of the objects placed thereon, using a camera system. The image and/or spatial data, e.g. one or more images and/or spatial information about the observed scene in any suitable form, specifically comprises depth information. Thus, acquiring 101 the one or more images and/or spatial data comprises acquiring 102 the depth information.

The method comprises obtaining 111 reference data representative of the patient support without the one or more objects placed thereon, in which this reference data comprises reference depth information. Thus, the reference data may be representative of the patient support without the one or more objects (e.g. patient, positioning aid, ...) to be detected and characterized in the following step.

The method further comprises determining 104, from the image and/or spatial data, at least one geometric attribute of the or each object (e.g. in the observed scene). This step of determining the at least one geometric attribute comprises detecting 105 one or more of the objects by, at least, taking the reference depth information in the reference data and the observed depth information in the acquired image and/or spatial data into account. Thus, the depth information and reference depth information may be compared, subtracted, contrasted or by any suitable technique analyzed in relation to each other, e.g. such that the observed depth information is compensated on the basis of the reference depth information. Then, the at least one geometric attribute is determined for the (e.g. each) detected object.

In acquiring 101 one or more images and/or spatial data by the camera system, the camera system may comprise one or more optical cameras, e.g. sensitive to one or more spectral bands in the visible (and/or infrared) range, e.g. monochrome camera(s) and/or color camera(s). The color camera(s) will be understood to refer to (a) color camera(s), without necessarily limiting to a specific choice of color components (e.g. RGB) or choice of their specific combination. It will also be understood that the "color" camera may also be (unintentionally or intentionally) sensitive to one or more infrared bands, possibly exclusively so, but also possibly in combination with visible light bands. The camera system is also not necessarily limited to "color" cameras with a relatively small number of color bands (e.g. Red, Green, Blue), but may also comprise (a) multispectral camera(s), e.g. which is adapted to quantify the light spectrum received at each pixel location more fully, e.g. by decomposition into a relatively large number of spectral bins (e.g. such that a substantial spectral image dimension is formed in addition to the 2D image coordinates).

Preferably, the camera system comprises or consists of a depth camera, i.e. to provide said depth information. For example, the depth information may be gathered in addition to in-plane image information, or, at least, three-dimensional (3D) information is acquired that is not trivially reducible to 2D data, e.g. not solely expressed in coplanar coordinates. For example, a 2D map (e.g. image) of depth, and/or a set of 3D points (e.g. a 3D point cloud) may be acquired. The set of 3D points may comprise (without limitation thereto) at least 32, but typically substantially more, points in space, i.e. that are not constrained to a same, single plane or on another trivial geometric surface, e.g. on a sphere, or cylinder, ..., or in other words, the 3D points are not trivially reducible to 2D data. The subject and/or auxiliary equipment, in the observed scene, may thus be characterized or characterizable (e.g. at least to some extent, to some level of accuracy, and/or within practical limitations) in three-dimensional space by the acquired depth information.

Thus, one or more depth images may be acquired 102, by a depth camera or by suitable alternative therefor. The depth information may be obtained in various possible forms, such as a (3D, exterior) surface geometrical and/or spatial characterization of objects in the camera's field of view, a point cloud of 3D points (sampled points on the surface of objects in the field of view), and/or an image (e.g. a matrix of pixel values, e.g. organized in rows and columns) in which each pixel has at least one value (e.g. a scalar pixel value, or one component of a vector value) associated therewith that represents a depth. For example, the depth value may be indicative of a distance from the camera to the sampled point in space corresponding with the image coordinates in projection space or, more generally, of a distance in a direction that points substantially out of the image plane. Such depth image may for example be obtained by a range camera, which may produce a 2D image that shows the distance to points in a scene from a predetermined reference point or plane (e.g. a focal point or other reference point).

For example, depth information may be obtained by stereo imaging, in which images are (e.g. substantially simultaneously) acquired by two (conventional, e.g. monochrome or RGB) cameras from different vantage points. Processing techniques known in the art may be applied to such stereo images (to the pair of concomitantly acquired images) to derive depth information therefrom, e.g. by parallax analysis. This principle can readily be extended to more than two cameras, which commonly may also be referred to as stereo imaging (i.e. "stereo" is not to be interpreted in the narrowest sense of using only two cameras). Likewise, the camera configuration is not necessarily limited to a setup for mimicking the human binocular vision (i.e. is not necessarily limited to the corresponding intra-ocular and intra-axial distances and/or inter-camera distances). For example, a depth image (e.g. a pixel map of depth values) may be obtained from the stereo camera system (generally, the multi-camera system) by stereo triangulation. An advantage of stereo (or multi-camera) depth imaging is that the needed device components may be easily obtainable and may be relatively cheap. For example, such system may be constructed using conventional (2D) cameras, or an existing (2D) camera may be easily upgraded by adding one or more further cameras. It is also noted that even a single camera fitted with a mirror and/or lens assembly may suffice to obtain data for the same point(s) in space from sufficiently spaced apart observation points and/or sufficiently different observation angles, e.g. splitting the image detector into multiple regions that receive light via different optical paths and/or using a switched mirror assembly to rapidly switch between different optical paths for sequential image acquisitions.

The processing required for calculating the corresponding depth pixel maps (i.e. depth images) is relatively simple, and can be easily implemented in software and/or hardware (potentially leveraging graphical processing unit, GPU, processing, and/or dedicated hardware, e.g. application-specific integrated circuits and/or field-programmable gate arrays). An advantage of stereo or multi-camera depth imaging is that the scene, e.g. the patient and/or equipment of interest in the scene, can be imaged without purpose-specific lighting and/or other direct manipulation. In other words, the scene can be imaged, and processed to determine the depth data (i.e. the third dimension in addition to the planar image coordinates), without actively interfering with or influencing the scene, i.e. essentially passively, except for, possibly, generic (general-purpose) lighting.

Depth images may also be acquired by active techniques. For example, a sheet of light may be scanned over the scene to image its reflection. From the shape and displacement of the imaged (line) reflection, the distance between the reflections (points on said line - e.g. every point in the image after fully scanning the scene) and a reference, e.g. the light source and/or camera can be relatively easily computed, e.g. using triangulation methods. Another type of active depth imaging that may be applied uses a structured light 3D scanner, which may be seen as a more intricate version of the light sheet triangulation mentioned hereinabove, with additional advantages such as requiring fewer (or none) physical displacement(s) of the camera and/or light source to fully qualify the depth values of the scene.

The depth information may be acquired by a time-of-flight camera, LIDAR or RADAR system. A possible advantage of time-of-flight camera depth imaging is that an image may be collected substantially instantaneously, e.g. without scanning a point, line, structured light pattern, laser, wave or generally any "active" (e.g. time-varying, e.g. scanning) analyzer over the scene. Other potential principles of depth imaging may include interferometric techniques (e.g. relying on coherent light), coded aperture imaging and/or possibly other techniques.

It will be clear that a diagnostic, e.g. medical, imaging and/or therapy session may impose constraints on the technique that is or can be used, or may create a preference for techniques with certain properties. For example, 'active' scanning techniques may preferably be avoided or limited to non-visible forms of light, particularly if a high intensity of light is needed, if the type of wave could potentially damage equipment or even the human body (e.g. laser light, e.g. particularly the eyes), and/or if a scanning (or other dynamic) pattern is needed that could be confusing, disorienting and/or fear-inspiring or unpleasant to the patient, might induce epileptic seizures in the patient or personnel, and/or could limit personnel in the performance of their activities. Thus, essentially passive techniques, such as stereo (or multi-) camera imaging might be preferable. Nonetheless, none of the mentioned techniques, including 'active' 3D imaging, are necessarily excluded.

In embodiments of the present invention, acquiring 101 the images may combine acquiring 102 depth information (e.g. comprising a pixel map with values representative of depth, a 3D point cloud and/or a parametric description of a surface, and/or in any other suitable form) with acquiring 103 optical 2D images of the subject and/or auxiliary equipment, e.g. photographs in the human visible spectrum, in the infrared spectrum, in (a) spectral sub band(s) of any of the aforementioned, and/or a combination of any of the aforementioned. Preferably optical 2D images are (co-)registered to the depth information, e.g. such that depth information and optical image pixel values correspond for the same image coordinate, or such that the registration information to relate image content in the depth image to image content in the optical image is determined by a suitable image registration method (as known in the art) and/or is predetermined and available (e.g. to a processor implementing the method) in another way, e.g. by a calibration procedure to relate the different camera views and parameters to each other.

It is to be noted that the camera system may be adapted to simultaneously (or concomitantly) acquire 102 the depth information and acquire 103 the optical image information. Depth images may thus be acquired as well as, preferably substantially simultaneously, more conventional monochrome, color (e.g. RGB) or multispectral images, in the visible and/or infrared spectral domain. For example, a multi-camera setup (e.g. stereo camera system) may obtain depth and optical (e.g. visual) image information simultaneously, e.g. by capturing the photographic image information and supplementing this with depth information obtained from processing said image information. Thus, a spatial relation between both types of image information is inherently known, e.g. the photographic and depth information are implicitly co-registered (or this relationship and/or a corresponding transformation is determined in a straightforward manner). Other depth camera systems, when alternatively used for acquiring the depth information, may also be suitable for gathering photographic information simultaneously, or (e.g. if not) may be combined with a further camera so as to be able to combine photographic information and depth information in a method in accordance with embodiments.

The camera system (or a part thereof) may be, for example, positioned generally above the subject support (e.g. above an imaging and/or therapy couch, patient table, ...) to observe the region where interactions with the patient in the imaging/therapy session will primarily occur from above, e.g. to observe the subject and/or any auxiliary equipment in an MRI scanner bore, on a CT patient table, on a radiotherapy couch, and/or in other such environment. It is noted that for some imaging and/or therapy environments, the patient (and/or auxiliary device) setup (i.e. in a preparation phase of the session) may take place near the interaction volume (of the imaging/therapy system), after which a simple, predictable and well-defined (e.g. actuated) movement can position the patient support along with the patient and/or any auxiliary equipment inside the target interaction volume, e.g. by translating the patient support without (ideally) any substantial relative movement between the patient support, the patient and/or any equipment item(s) set up for the procedure. In such scenario, it will be understood that the camera system may be positioned generally above the patient support where it is in its (typically easily accessible) configuration for preparing the patient and procedure (e.g. assisted by an operator). The camera system may also be positioned above the patient support in a configuration thereof where the imaging/therapy procedure is intended to be performed, e.g. when the system is not configured to perform any substantial automated translation between the preparation and execution phase of the session (for example, as may be commonly the case in radiotherapy environments). It will also be understood that the camera system may comprise various components, e.g. multiple cameras and/or sensors, and/or may be configured for a relatively wide view, such that both preparation and execution positions (if substantially different at all) of the patient table can be adequately viewed and analyzed using the same static camera setup.

Thus, at least one camera (which generally may refer to any device for gathering depth information and/or optical image information) comprised in the camera system may be positioned above the patient support and oriented toward the patient support such as to be able to acquire depth (and optionally further optical) information in a field of view sufficient for observing the patient and/or equipment to be used in the patient's vicinity, from an overhead perspective. Alternative positions and/or orientations of the camera axis (axes) are not necessarily excluded. Furthermore, it will be understood that the terms 'above' and 'overhead' do not (necesssarily) require an exact vertical position or alignment of the camera(s) above the patient, nor a camera axis that is directed (exactly) vertically downward. For example, in an MRI environment, it may be advantageous to view the patient table at a relatively shallow angle (without limitation thereto) and/or from a point (camera position) relatively distant from the patient's center of mass (e.g. distant in horizontal projection), e.g. 1 m or more. Thus, interference between the camera system and the MRI system may be avoided, or at least reduced, e.g. by positioning the camera(s) near or at a magnet bore flange, or even farther away, e.g. on a wall of the scanner room or even outside the scanner room (e.g. observing the patient table through an observation window).

The method 100 comprises determining 104, from the camera images and/or spatial information acquired via the camera system, at least one geometric attribute of the patient (and/or one or more body parts of the patient) and/or the auxiliary equipment. The at least one geometric attribute may for example be calculated by processing of a segmentation, a set of identified pixels corresponding to the object of interest, and/or another suitable representation of the (e.g. each) detected object.

The detection of individual objects (which may include the patient) and processing each detected shape or volume such as to determine representative geometric attribute(s) thereof, clearly has advantages when applied in a patient and/or device preparation phase of an imaging/therapy session. By detecting different objects in the scene and documenting the spatial configuration of each object and/or the spatial relations between the objects in a more representative and/or reproducible form, an accurate reproduction of the same setup and/or an accurate documentation of the setup for future reference can be achieved.

The geometric attribute or attributes that are determined for the one or more objects, such as the patient, body part(s) of the patient, and/or one or more equipment items, e.g. positioning aids, may be implicitly obtained by the step (discussed further hereinbelow) of detecting 105 the object(s). For example, a detection algorithm may be applied that implicitly determines characteristics of the different object(s), e.g. centroids or other such measures indicative of location (and/or orientation, and/or other geometric properties). However, the geometric attribute(s) may also be determined, e.g. for each object (e.g. independently), based on the result of said detection 105, e.g. by an explicit calculation thereof, e.g. based on a spatial clustering and/or segmentation output.

The detection process is preferably agnostic, e.g. preferably does not take a specific geometric model of the/each (type of) object into account, but detects objects by their isolation (in space and/or time, e.g. the first appearance of different objects in the scene being spaced apart in time), separation, distance with respect to each other, (zero or limited) interfacial surface, volume or contours, and/or other such generic properties, i.e. properties that may generally apply to any object of interest. However, embodiments are not necessarily limited thereto. For example, the patient or body part(s) of the patient may be detected in a different manner than the/any equipment item of interest. Thus, optionally, a step of determining whether a detected object corresponds to a living subject, e.g. the patient, or to an inanimate object, e.g. a postioning device, may be included, or such differentiation may be implicitly included in the method of detection. Some examples are provided hereinbelow, without limitation thereto, e.g. to distinguish a patient from an inanimate object based on motion and/or body heat (infrared radiation), and/or simply by a substantial difference in volume, surface, contour length and/or, generally, physical dimensions. This allows for (a) different geometric attribute(s) to be determined and/or presented and/or stored for the patient than for, e.g., a positioning aid device.

It may be particularly advantageous to detect different equipment items in substantially the same or similar manner, which allows the method to be applied to a wide range of such potential objects and/or without requiring prior specification of an object model (or other object class descriptor) and/or without needing modification of each such equipment item (e.g. by inclusion of physical identification markers, tags, and/or other means of facilitating automatic detection on/in the object). The geometric attribute(s) are preferably unambiguously and/or robustly defined (e.g. automatically computable) for any equipment item, e.g. may preferably relate to properties that are detectable in a reproducible manner from repeated applications of the method, e.g. from segmentations as discussed hereinbelow in different imaging and/or treatment sessions and/or in different environments (scanner or therapy systems/rooms). However, the geometric attributes associated with the patient may be different from those associated with an equipment item (or may be the same). The attribute(s) determined for each detected equipment item may be preferably the same (e.g. of the same nature and/or determined by the same algorithm/process). By treating all objects of interest in the scene essentially equally, difficulties in recognizing different types of object, possible issues with incorrect identification and/or a need for intricate modeling or means of specific identification of different objects can be advantageously avoided or reduced. Alternatively, some distinction may be made in processing different types of object, e.g. based on broadly defined and/or robustly detectable characteristics, and/or a simple and robust characteristic may be used to identify a detected device from an easily maintainable library of devices (or a specification of the devices used for the specific session). For example, an object (or type of object) may be identified by its volume or projection area, such that the method can be easily configured to recognize a specific device (type) by a simple and quick calibration or the entry of one (or a few) identification property (properties).

By determining geometric attributes of objects, a spatial relationship between these objects can be clearly determined, e.g. to facilitate the positioning setup process (for example, when live updates of the attributes are provided as feedback to an operator during setup) and/or to support quality monitoring and documentation processes. The geometric attribute may generally comprise any value that is indicative of a spatial configuration of the object, e.g. of position, an orientation, a deformation, a shape, etc..

The geometric attribute(s) may comprise simple summary statistics and/or other representative values, e.g. a center of mass (or at least, of volume and/or of area or surface as observable in the camera view), a measure of orientation and/or a measure of mass, volume, and/or area.

For example, the at least one geometric attribute may comprise a position, in one, two or three directions (e.g. along non-colinear and/or orthogonal coordinate axes). For example, the position may correspond to a center of the patient and/or equipment item, such as a geometric center, a center of mass, a centroid, or generally any point that can be used to represent the position of the object (the patient, a body part of interest of the patient, an equipment item, ...) as a whole, preferably in a unambiguous and reproducible manner. For example, the position (and/or orientation) of the patient (i.e., generally, a human or animal) and/or of one or more devices (equipment items) used in the preparation workflow may be computed.

The physical position of the object is not necessarily fully qualified by the position included in the at least one geometric attribute. For example, the position may be defined along a single axis, e.g. a longitudinal exis of the patient table or an axis of the diagnostic imaging system or therapy system, may be defined with respect to a plane, e.g. the horizontal, the plane of the patient table or a reference plane of the imaging/therapy system, or may be fully qualified, e.g. by three coordinates in a (e.g. Cartesian) coordinate system. The position of the (or each) device may be limited to a discrete value from a set of known patient support indices, e.g. a patient support index as commonly used in radiotherapy (without limitation thereto), e.g. a discrete index taking a value from the ordinal set F6, ..., F1, 0, HI, H2, H3 (e.g. as discussed in the background section hereinabove). It will be understood that embodiments may equivalently use an integer value to represent such discrete label, or a different scale (and/or labels) may be used. It will also be understood that a real-valued coordinate may also be used instead of a discrete index, or a suitable approximation thereof (fixed point precision, floating point values, ...). This discrete position information (e.g. a longitudinal position index) may also be extended, e.g. by further coordinates (e.g. in the lateral direction and/or vertical direction), which may be discrete or not. The discrete (e.g. longitudinal position) index may also be complemented with more precisely defined information (e.g. a real value position) for the same direction.

As an example, a position may be represented (e.g. for display and/or storage purposes) by an annotation such as "pos: F2, x:-12.1, y:-25.3, z:4.2" in order to provide easy access to the approximate position as well as more precisely defined coordinates. This example is not limitative; arbitrary units were used for illustrative purposes; the illustrative label F2 does not necessarily correspond to the illustrative coordinates in any real world context. The redundant information may be used as needed, e.g. by a human and/or machine user. For example, a human user might rely on the index label for quick reference, whereas a processing algorithm might use the detailed position coordinates for post-processing the recorded data.

Likewise, the at least one geometric attribute may comprise an orientation, e.g. (without limitation thereto) expressed as one, two or three angles, e.g. with respect to suitable coordinate axes or reference planes. The orientation may be fully qualified, i.e. in a form that uniquely identifies an orientation vector in 3D space, or with respect to only one axis or with respect to only one plane.

A geometric attribute(s) may be determined by fitting a 2D or 3D shape to the (e.g. each) detected object. For example, a rectangle, ellipse, rectangular cuboid (or prism), ellipsoid, and/or another simple geometric 2D or 3D shape may be fitted to the detected surface/volume/pixels to summarily represent the detected object in the scene, e.g. placed on a patient table. The length and width of a fitted rectangle, the major and minor axis of a ellipse, and/or other such parameters of a fitted shape (in 2D or 3D) may be used as geometric attributes representing the orientation and/or scale of the object. Instead of fitting a shape (e.g. by optimization), similar parameters, e.g. a maximum, minimum or mean width (and/or length and/or height), may be determined along one or more predetermined axes, e.g. along the longitudinal axis of the subject support (and/or the lateral and/or vertical directions perpendicular thereto). This is less computationally demanding than applying a shape fitting, and may be sufficiently informative, accurate and/or reproducible for at least some use cases, e.g. where (relatively large) changes in orientation are not expected,

Other examples of geometric attributes include a total estimated volume, a total estimated surface area (e.g. in the camera projection view or taking surface angulation into account) and/or a circumference (e.g. of the contour in the camera projection view). The geometric attribute may also comprise a representation of the contour in some suitable form, e.g. a lasso representation.

The geometric attribute may particularly comprise a representation of a centerline of the detected object, as discussed further hereinbelow in detail.

The method 100 comprises obtaining 111 reference data representative of the patient support without the object(s) (which is/are present when acquiring 101 the data) placed thereon. The reference data comprises reference depth information. Furthermore, determining 104 the geometric attribute(s) from the image and/or spatial data comprises detecting 105 that object or those objects. This detection uses at least the reference depth information in the reference data and the depth information in the acquired image and/or spatial data, but may also take other information into account, e.g. optical (e.g. 2D) image data, such as (an) infrared, color and/or monochrome image(s) concomitantly acquired with the depth information.

It is one of the advantages of using depth data that any object with a nonnegligible volume, regardless of its specific shape or specific object (or object type) properties, can be easily detected. However, a paper sheet on the subject support would typically not be detected in view of its thin profile (i.e. not causing a significant change in depth along the corresponding camera depth trace paths). For example, an operator may typically use handwritten or printed notes to keep track of the procedure(s) being performed. Such notes can easily end up on the patient table (at least temporarily during the preparation phase), but would not easily interfere with the detection process 105 when taking the depth information into account. The sensitivity of such depth-based detection 105 may also be adjustable, configurable or tunable, such that a lower threshold (e.g. for depth difference or absolute depth difference, or any suitable equivalent, e.g. a suitable metric/norm distance) may be defined to reject other potential nuisance or interfering (thin) objects automatically as well, e.g. a smart phone or tablet device. Such threshold (explicit or by an equivalent adjustable algorithm parameter) may also be effective in avoiding false detections due to small deformations of the patient table, e.g. when the patient is place on a cushioned subject support. It will be understood that an upper threshold may optionally be applied as well, e.g. to ignore untypically large/high objects, such as the (e.g. temporary) presence of an operator in the scene.

The detection 105 of the object(s) of interest may use, implicitly or explicitly, a background subtraction or compensation, or at least takes the reference depth information into account to detect the object(s) of interest. For example, a reference image or other reference data (particularly including depth information) representative of the subject support (patient table) without the object(s) to detect, e.g. in the same imaging/therapy system and/or environment of intended use of the method, may be used to detect the object(s) of interest. Thus, the reference data, e.g. reference image(s), may include a representation of the subject support without a patient and without positioning aids or other devices of interest (i.e. to detect by the present method) thereon (or more generally: in the represented reference scene), but the reference data may also represent an intermediate state in which some object(s) are already included in the reference data, but at least one further object is yet to be detected and characterized in a next iteration, as discussed in further detail hereinbelow.

The object detection process may be as simple as (or may comprise) subtracting the reference data (e.g. a reference depth image or reference depth data, e.g. organized per camera observation ray) from the presently acquired 101 image/spatial data and clustering the non-zero results (possibly after taking a predetermined threshold into account) to detect (e.g. the shape, the volume, the pixels and/or other descriptive spatial reference of) an object currently present in the scene that is not present in the reference scene, and/or to detect a plurality of separate entities (objects). Differences may be processed in absolute value (or equivalent, e.g. squared difference, ...), or may, alternatively, be considered only when the sign of the difference indicates an object above (closer to camera) the reference (e.g. empty) patient support.

Thus detecting 105 the object(s) may comprise determining 110 points for which the corresponding acquired depth information differs substantially from the corresponding reference depth information, i.e. where the observed point in space does not fall on (or underneath) the surface(s) characterized by the reference data (e.g. do not coincide with the subject support).

The obtained 111 reference information may characterize the patient support in the form of one or more (e.g. depth) images of the table without the patient and without the equipment (still) to detect. Alternatively (or additionally), the reference information may comprise a geometric model of the patient support computed in the camera coordinate system, or another type of model to relate the camera coordinates and/or image content received by the camera system (which may include depth information) to the subject support (patient table), e.g. in a condition where no patient or (further) equipment items of interest are present. Thus, from the (image and/or model) reference information, any arbitrary object placed on top of the patient support, e.g. one or more positioning devices (or other relevant objects) and/or the patient, may be detected, using the depth data and possibly further camera imaging information (e.g. a monochrome or color digital photograph), e.g. by comparing the depth data of a point to the reference and computing whether the point is above the respective point in the reference configuration or not. Hence, any arbitrary mass distribution placed on top of the reference configuration can be detected and its geometric attributes can be computed, without necessarily requiring specific assumptions about the objects as such (e.g. different geometric models for specific types of object that could be encountered).

It will be understood that a segmentation and/or clustering used in the detection 105, in accordance with some embodiments, may receive such reference-compensated data as input, may take the reference information as additional input to the camera observation data (e.g. as further input for a trained machine learning model) or may be applied on the camera observation data as such. For the latter, the result of the detection (e.g. segmentation) may be compensated for the reference information, e.g. by rejecting segmentation segments that correspond to identified corresponding elements in the reference, e.g. the table and/or static or irrelevant equipment (for tracking purposes). It will also be understood that an iterative procedure is not necessarily excluded, e.g. alternating a segmentation (or other type of object detection step) with a background content removal step.

Obtaining 111 the reference data may comprise receiving a current position of the subject support (e.g. height, longitudinal and/or lateral position, e.g. the three coordinates of an automated table translation system), and calculating or updating a (e.g. parametric) three-dimensional geometric model of the subject support. The current position, which optionally may also include, if relevant, orientation or other geometric parameters (e.g. orientation angle or angles), may be provided by a control system of the patient support. This current position information may thus be used as input to an image and/or data processing algorithm. Alternatively, this position (generally, the spatial configuration of the subject support) may be computed from the acquired image and/or spatial data directly, e.g. when a direct input of the position information is not available or not easily obtained. This position information may be used in combination with other reference information, such as a geometric model of the patient support as such (which may use the position or spatial configuration information as parameter) or reference image information, e.g. reference images (2D and/or 3D) of the patient support indexed by or in another way defined as function of the specific position (or spatial configuration in general). The reference information may comprise a geometric model of the patient support, preferably defined in the camera coordinate system, in which case the current position of the patient support can be used to evaluate the model for the specific patient and procedure at hand. The reference information may comprise a set of images, e.g. including depth images, used as reference for a plurality of different positions. The set of images may be interpolated if needed to the position received as input, for example interpolation by nearest neighbor approaches, or a more detailed interpolation may be used (e.g. linear interpolation, non-linear interpolation, ...). It will be understood that if the set is sufficiently comprehensive and/or the position of the table in use of the method is always limited to well-defined discrete positions (e.g. steps of a stepper or indexed motor system), interpolation may not be necessary.

Detecting 105 the object(s) may comprise performing an image segmentation and/or, more generally, a spatial segmentation and/or clustering, e.g. of image data and/or data in the form of a 3D point cloud and/or in another form of representation of 3D data. The segmentation and/or clustering may preferably use a naive or agnostic algorithm, i.e. that does not rely on prior knowledge about the specific object(s) to be detected, but may take some generic assumptions into account that reasonably apply to essentially any possible object of interest, e.g. to all equipment items, such as positioning means, that could reasonably be included in the observed scene. As already indicated, this detection may be relatively simple, i.e. when the reference information is taken into account, e.g. a single object can be readily detected by background compensation, subtraction or the like in a depth image with high sensitivity and relatively low risk of false detection. Multiple objects can be detected by using suitable segmentation and/or clustering techniques.

The method may be used in a sequential manner, e.g. to advantageously reduce complexity, increase robustness and/or improve accuracy. Specifically, in each step, a new object may be added to the scene (e.g. by an operator) and a corresponding change with respect to a previously established scene (in a previous step) may be detected. Thus, detecting 105 the object is relatively simple: objects that would be abutting, overlapping, or otherwise difficult to distinguish when presented simultaneously can be easily discerned by focusing on each incremental update in the constructed scene, in which a previous object or objects remain essentially static and a (preferably) single new object is introduced and moved in the scene until correctly positioned.

It will be understood that, for example, a next step can be triggered by an action of the operator, e.g. a button press, voice instruction, or other suitable user interface interaction, or detected by processing, e.g. a sudden substantial change in overall scene content while the object that was being tracked remains essentially stationary could be used to signal that a new object has been added and a recently acquired scene (image data, 3D data, model abstracted therefrom, or similar representation of the acquired image and/or spatial data at a recent point in the past) may be used as a new reference while tracking the new object. As another example, when the detected object(s) remain stationary for a predetermined time, it may be assumed that the operator intends to switch to a new step, in which a further object (e.g. the patient or another positioning aid) will be positioned. Thus, this may also be used as a trigger to store the present scene content for use as reference and start a new iteration block for detecting another object or objects.

In other words, in the method 100 in accordance with embodiments of the present invention, the steps of acquiring 101 the image and/or spatial data and detecting 105, based on that data, at least one object may be repeated 114. After each iteration of this repetition 114, e.g. after determining 104 the geometric attribute(s) of the newly detected object(s), the reference data may be updated 113 to include the detected object(s) (in that iteration) such that the updated reference data represents the patient support with the object(s) that have already been detected up to the completed iteration. Thus, one or more further objects can be detected in a next iteration. Even though such sequential approach may be particularly robust, simple, accurate and/or efficient, embodiments are not necessarily limited thereto. However, in many situations, the workflow of an operator may already be organized to add different objects to the scene in a sequential, stepwise manner. For example, a knee support may be positioned on the table at its intended location, after which the patient may take place on the table (and knee support) while the operator ensures that the position, the orientation and, generally, the spatial configuration of the patient conforms to any applicable requirements. Other examples can easily be found where the order in which objects are placed in the scene is determined by necessity, by common sense or by reasons of efficiency.

However, various approaches can be applied to detect and distinguish several objects that are presented at once, such that a sequential approach may also be avoided. For example, a method in accordance with embodiments may be adapted to allow multiple objects to be detected and isolated with respect to each other even if none of these objects is represented in the background data. In other words, a method may be applied sequentially to detect one new object in each step, adding this information to the reference data at the end of each step, but may also be adapted to detect multiple objects relative to the reference data (simultaneously) in a single (or fewer) step(s). Therefore, the approaches discussed hereinbelow to segment and/or cluster the data such as to identify a plurality of objects, e.g. in which each object is identified separately, may be applied as alternative to the sequential approach discussed hereinabove, or may be used in combination with such sequential approach.

It is noted that the quality of a segmentation, particularly one that is agnostic (i.e. not based on explicit models of objects), may be increased by obtaining additional data per pixel. For example, a segmentation of a color image may be easier (e.g. performed more efficient) and/or of a higher quality (e.g. higher accuracy) than of a monochrome image. Likewise, depth information, e.g. expressed as a depth value per pixel, but not limited to such form of representation, and possibly in combination with other scalar values per pixel (e.g. a depth or Z value in combination with an image intensity, different colors, spectral components and/or other such properties, for each pixel) may allow a fast, efficient and accurate segmentation. Since depth information is available, this may advantageously be included in a segmentation and/or clustering approach to improve the ease of detecting distinct objects in space.

Isolated, or minimally connected (e.g. with respect to a predetermined threshold), objects may be detected by clustering 3D point data, image data (e.g. segmentation) or a combination of both. Image segmentation and/or clustering algorithms suitable for use in a method in accordance with embodiments are generally well-known in the art. The segmentation and/or clustering may use a "classical" computer vision algorithm and/or an approach based on artificial intelligence, machine learning, deep learning and the like. Examples of classical (declarative) algorithms include simple (e.g. pixel value) thresholding, histogram-based methods, clustering (e.g. K-means and many others), compression-based methods, edge-based methods (e.g. edge detection), region-based methods (e.g. region growing, watershed transformation), differential equation and/or variation based algorithms, graph partitioning (e.g. Markov fields, a posteriori partitioning, expectation maximization, ...), and/or multiscale approaches. This list of examples is not exhaustive, and combinations of such methods may also be considered.

It is also noted that the combination of depth information with more conventional 2D image information may offer the advantage of easily and accurately detecting isolated or only slightly touching objects (based on depth) while also allowing different objects that are touching or overlapping to be distinguished by their visual appearance, e.g. their color, intensity, and/or optical properties.

In this context, it is also noted that inclusion of infrared image information, e.g. in addition to depth information and/or visible spectrum image information, can be particularly useful to distinguish inanimate objects, such as equipment items, e.g. positioning tools, from living beings, e.g. the patient and/or body parts thereof. Thus, a particularly advantageous embodiment may use depth information in combination with infrared information, optionally further combined with image information in the visible spectrum.

The image and/or spatial data (e.g. depth data and optionally further image data) may also be acquired as a live stream or, generally, in a dynamic manner. Since the information about the observed scene can thus be dynamic, e.g. updated at a predetermined (non-zero; not necessarily constant) refresh rate, motion-based segmentation and/or clustering techniques may also be used, e.g. as stand-alone or in combination with any other segmentation or clustering technique. For example, this may be particularly useful for detecting a background, e.g. the patient table, the scanner/therapy room, and/or other irrelevant static scene content. Thus, a dynamic stream of image/spatial data may be acquired 101 and analyzed to determine the reference data discussed hereinabove, e.g. determining the reference depth information (and/or other reference image information) from acquired data corresponding to an earlier point in time, e.g. when a 'save state' trigger was provided by an operator (or inferred automatically from the data) to indicate that one or more new objects will be added to the scene. For the sake of completeness, it is noted that a buffer of data may be maintained, such that such trigger can be generated at some time after the point in time representing the reference state, e.g. when a new object is detected in the scene, a suitable previous point in time may be determined (e.g. searched in the buffer) for which the data represents a suitable reference state without the new object.

Even though the purpose of the procedure in which the method in accordance with embodiments is used may require the patient to lie still, it will be understood that some residual motion typically cannot be avoided (e.g. breathing and/or other involuntary movement). This can be exploited to improve the detection of the patient or his/her body parts as such. Likewise, while positioning means or other equipment may be intended to remain immobile during the procedure to be performed, it will be understood that the present method may be applied during a session preparation, in which an operator adjusts the position, orientation and/or other spatial properties of such devices in the scene. Therefore, motion analysis may be used to detect objects for which the position or spatial configuration is to be monitored, since image components that substantially change in the scene are either equipment items of interest, the patient or (optionally) the operator manipulating the scene. Motion of the patient can be distinguished from motion of such equipment items by a substantial difference in their dynamics.

If the operator is explicitly taken into account (e.g. to be explicitly ignored in processing of the scene), the dynamics of motion of the operator can also be easily distinguished from equipment items as well as the patient. For example, objects that move outside the field of view of the camera system, or at least show a dynamic tendency of movement between the periphery of the scene and the center of the observed scene (or between the edge and positions of detected objects and/or the patient in the scene) may be identified as personnel and excluded from the detection process. By optionally applying motion tracking, e.g. based on optical flow or other such techniques, the operator may, once identified, be tracked throughout the procedure of setting up the patient, e.g. may be easily excluded from consideration if detected in the analysis of previous images (i.e. previously acquired 101 image/spatial information) obtained in the same session. Infrared data, as discussed hereinabove, may also be used in combination with motion analysis to further improve the detection.

The depth data may be processed by generic segmentation algorithms, e.g. may be treated as merely an image of a value without taking the meaning of this value as depth into account. For example, the image may be represented and processed as a vector-valued image, in which one vector component may thus represent the depth or third dimension data. However, the depth information may also be taken into account more explicitly, e.g. processed in a manner that is not necessarily identical to the processing of other image components (if taken into account at all), e.g. color components. Depth data that is represented as a cloud of 3D points may be processed by point clustering or another 3D technique to detect objects, such as partitioning 3D shape or surface data into a number of minimally connected parts. For example, a joint optimization of cost function components and/or constraints may be used to identify objects in the 3D data. Illustrative cost function components may include a measure of correspondence between the segmented model and the observed 3D data (e.g. a mean squared difference or other suitable measure, as is generally known to be used in such optimization problems), the number of partition segments (e.g. to favor fewer objects in order to reduce potential overfitting) and/or lengths, surfaces or other such measures that are indicative of the intersection of (or the geometric interface between) different objects, and/or a measure of distance between objects, without limitation thereto. Other 3D clustering, segmentation and/or similar processing techniques may be applied, as generally known in the art, including the application of artificial intelligence techniques.

It is noted that in a particularly simple embodiment, a segmentation or similar detection of separate objects of interest may even be avoided or may be implemented in a very rudimentary fashion. For example, merely contrasting depth data (and optionally conventional image data) with the reference depth data (and optionally image data) may be sufficient to identify a single object that corresponds to the difference in the observed scene with respect to the reference scene.

The method may comprise repeatedly 106 performing the data acquisition 101 and characterization of the scene and/or objects therein by determining 104 the at least one geometric attribute and presenting the updated information to an operator, e.g. in a graphical representation of the scene showing the at least one geometric attribute. For example, the determined at least one geometric attribute may be represented in a visual form to the operator, e.g. in an overlay on at least one (e.g. a most recent) camera image as acquired 101. Examples include the presentation of detected objects, e.g. determined by the segmentation, in overlay on a (e.g. visible spectrum) camera image, e.g. in a translucent color overlay or as contour lines, and/or a graphical representation of the geometric attribute(s), e.g. indication of center points, center lines, fitted shapes (e.g. a fitted box, circle, ellipse, etc.) and the like on representative locations in the image. The representation may include numeric values of the attributes or display elements in another suitable form. For example, a distance between a detected position and a reference position (from a specification or earlier stored procedure) may be shown in a numeric form or as a bar, e.g. similar to a progress indicator. It will be understood that many alternative forms of presentation can be envisioned as well.

Therefore, the method may comprise a step of presenting 107 the at least one geometric attribute to an operator, e.g. via a display monitor or other suitable human interface device. Preferably, such feedback to the user may be dynamic, e.g. by repeating 106 the detection and attribute calculation referred to hereinabove. For example, a center point, a centerline, an axis or axes of orientation, a bounding box and/or other such visualizations of the (determined geometric attributes of) object(s) may be shown on an image (or geometric model) of the scene.

The method may also comprise storing 108 the at least one attribute, e.g. in a data storage, for future reference. For example, this allows the positioning of the patient and relevant equipment to be reviewed at a later instance, e.g. for quality assurance and/or workflow documentation. This also allows the stored 108 information to be retrieved in a future session, particularly in which the same (or similar) positioning of the patient and/or equipment is desired. For example, such information may be stored after a phase of guided positioning and/or orienting (generally, spatially configuring) of objects has completed, so as to make the information available for future reference, or the stored information may be updated throughout the procedure. The information may, for example, without limitation thereto, be stored as annotation(s) and/or metadata in (e.g. diagnostic) image data acquired by the imaging and/or therapy procedure, e.g. in (a) DICOM header(s) and/or a PACS system.

The method may also comprise retrieving 109 the at least one attribute, i.e. stored 108 in a different (e.g. previous) session, for comparison purposes, if such previously stored information is available. The different session does not necessarily relate to the same type of imaging and/or therapy procedure. For example, without limitation thereto, information about the positioning and spatial configuration of the scene in general may be recorded in an imaging session, and used to replicate the scene (with sufficient and/or maximum achievable fidelity) in one or more therapy sessions, e.g. radiotherapy sessions, and/or in an imaging session using a different imaging modality.

The presentation 107, e.g. for guiding an operator in configuring the scene, may take such retrieved 109 data into account, e.g. by simultaneously displaying the present scene configuration and the previously recorded scene configuration, by showing one or measures representative of a difference between said scene configurations and/or by another guidance approach to assist the operator in comparing the previous and present conditions, to assess the deviation and/or to reproduce the previous configuration. Procedure and/or patient information may also be retrieved that is not explicitly stored in a different session. For example, (a) procedure guideline(s), a session planning, a prescription or a similar specification of the intended imaging and/or therapy session may define the type of, number and/or positions of patient support devices (and/or other relevant auxiliary equipment items) for use in the session at hand (possibly accompanied by information on the intended patient pose, position, orientation, etc.). For example, such procedure information may contain the positions to be used for positioning the devices in the patient preparation guided by the method.

To illustrate embodiments of the present invention, a possible workflow for preparation of an imaging exam or therapy session is described hereinbelow. For the specific procedure, e.g. with the patient support in a known position, the method may comprise obtaining the relevant reference information. In other words, the empty state of the patient support is determined (e.g. the "empty" state as it would be observed in the image and/or other spatial data acquired by the camera and/or, generally, from the camera viewpoint or viewpoints). A geometric model and/or reference camera images may be used to determine a representation of the (empty) patient support for its current position, in which, optionally, a received current position of the table support may be used. The camera system may alternatively be triggered to acquire this reference information on the spot, e.g. before the patient and/or relevant equipment is placed.

An operator may position the patient and/or any required equipment items on the table. The method may optionally present information to the operator, e.g. via a display or other interface device, e.g. based on retrieved procedure and/or patient information, to aid the operator in selecting the positioning device(s). This presented information may also comprise information stored by a previous use of the method, e.g. positioning information of the patient and/or any device(s) as recorded in a previous session for the patient.

For each equipment item placed by the operator on the patient support, the camera system may acquire image data, e.g. a depth image (or generally 3D data) and/or other (optical) image(s), e.g. in the visual and/or infrared range.

The method may then determine which pixels and/or spatial data in the acquired data correspond to points in space above the patient support, which may be done by using the aforementioned reference data representative of the empty state of the patient support. Optionally, this may take further image data, e.g. in the visual range, into account as well, e.g. to increase the accuracy of the identified pixels and/or data points that are not attributable to (only) the reference state. For the selected data, e.g. the pixels in the depth image that are determined to be elevated with respect to the table, a set (e.g. point cloud) of corresponding 3D coordinates, or an equivalent spatial representation, may be determined. Then, a clustering algorithm may be applied to the points in the determined point cloud (or equivalent processing when the identified points and/or regions in space are in a different spatial representation), so as to compute one or more (e.g. point) clusters. Each cluster can be represented on a visualization provided to the operator, e.g. on the camera image (or a specific image component thereof; or a synthetic image computed from the camera image data). For example, each point cluster may be marked by a color overlay (e.g. each a different color) on a camera observation image.

The geometric attribute(s), such as a centroid and/or principal orientation axis (or axes), may be calculated for each point cluster. To each cluster, a class identification tag (ID) may also be assigned, such as to allow different objects to be distinguished (e.g. without confusion or interchangement) throughout the procedure and/or in later use of the information. This may be based on spatial properties, e.g. preferably substantially invariant properties, such as a generic shape and/or volume descriptor, or merely a number assigned by the order of detection, or any other suitable strategy.

The geometric attribute(s) may also comprise a patient support index calculated from the detected position (e.g. cluster centroid), e.g. a closest patient support index value (which may be a discrete scale on the longitudinal axis). This patient support index may be particularly useful for documenting and storing the position of devices in a quality control system, e.g. a radiotherapy quality system. For example, the closest (discrete) index to the cluster center of mass may be computed. This index may be displayed for the convenience of the operator and/or stored for later reference, e.g. to document the workflow.

This information, e.g. the patient support index and/or other geometric attribute(s), may be presented to the operator, e.g. in a numeric form or graphical representation on said camera observation image, e.g. displayed in (substantially) real-time to the operator to guide him/her, e.g. in applying corrections to the positioning, orientation and/or, generally spatial configuration, of objects (incl. possibly the patient) in the scene.

If several devices are to be positioned, the devices may be handled consecutively. This may avoid confusion and may improve the stability and accuracy of the method. Instead of detecting multiple clusters simultaneously, by only adding a single item at a time (including potentially the patient), an algorithm in accordance with embodiments can easily track (incremental) changes in the setup. After confirming the correct placement of an object (e.g. a device or the patient), the operator may proceed to a next item, at which time the method may use the present (confirmed) state to determine a new reference for the next phase. Thus, for example, a depth image representative of the reference state (e.g. initially the empty support) may be updated in accordance with a current image, e.g. including a recently placed object, such that changes indicative of a new object added to the scene can be easily detected and processed. Alternatively, a geometric reference model (initially representative of the 3D points of only the table support) may be updated to include the 3D coordinates (or other spatial descriptor) of the object(s) as were positioned in the previous step(s) as well.

In a second aspect, the present invention relates to a device for assisting in the positioning and/or orienting of a plurality of objects, including a subject and/or at least one auxiliary equipment item, on a subject support in an imaging and/or therapy session. Fig. 2 schematically illustrates a device 10 in accordance with embodiments of the present invention. For example, the imaging and/or therapy session may comprise a magnetic resonance imaging session, a computed tomography imaging session and/or a radiotherapy session. The device may be adapted to detect positions, orientations and/or other spatial properties of the patient and/or the auxiliary equipment and/or to detect spatial relationships between the subject and the equipment and/or between different equipment items. Thus, the device may be adapted to assist in patient and/or equipment positioning (and/or orienting) in a preparation step of the imaging and/or therapy session.

The device 10 may comprise a processor, data storage memory, input(s), output(s), a user interface and/or other means generally known for performing a method as discussed hereinabove, e.g. when programmed and/or configured accordingly. Thus, the device may comprise a computer and a computer-program product in accordance with embodiments of the present invention (i.e. adapted to be executed by the computer). Additionally or alternatively, the device may comprise hardware specifically designed and/or configured to perform a method in accordance with embodiments of the first aspect of the present invention, e.g. comprising an application specific integrated circuit and/or configured field-programmable gate array to perform a method in accordance with the first aspect of the present invention. The device 10 may be comprised in a workstation or in an imaging and/or therapy system.

The device 10 comprises a camera system 11 for acquiring image and/or spatial data of the subject support having at least one of the objects placed thereon, in which the camera system is adapted for acquiring depth information. The camera system may comprise at least one camera arranged above the subject support such as to obtain a two-dimensional and/or three-dimensional overhead view of the subject support. The camera system may comprise a depth camera. The camera system may comprise at least one optical camera for acquiring a two-dimensional image. The camera system may comprise a plurality of two-dimensional optical cameras to acquire (e.g. substantially simultaneously) two-dimensional images from a plurality of different vantage points. Thus, the camera system (and/or processor) may be adapted to determine the depth information from the two-dimensional images, e.g. by applying an algorithm for depth inference from stereo or multi camera images.

The device comprises a processor 12 and an output 13. The processor 12 is adapted for obtaining reference data representative of the subject support without the at least one object placed thereon, in which this reference data comprises reference depth information. The reference data may comprise a three-dimensional geometric model of the subject support and/or at least one depth image of the subject support.

The device may also comprise an input 14 for receiving a current position of the subject support from an automated subject support actuation system, or the processor may be adapted for determining such current position from the acquired image and/or spatial data, e.g. by fitting a model of the subject support to the acquired data. Thus, the processor may be adapted for determining the reference depth information based on the current position and a three-dimensional geometric model of the subject support, e.g. defined as function of its position, and/or based on the current position and a set of reference depth images of the subject support, e.g. corresponding to a plurality of different possible positions.

The processor 12 in adapted for detecting the object(s) (on the support) by at least taking the reference depth information in the reference data and the depth information in the acquired image and/or spatial data into account. The processor 12 is adapted for determining at least one geometric attribute of the or each object (on the support), and for outputting, via the output 13, the at least one geometric attribute in a suitable form.

Without limitation thereto, the determined geometric attribute(s) may comprise a discrete subject support index, selected from a predetermined discrete ordinal set of subject support indices, to identify a position of the detected object associated with said at least one geometric attribute along a longitudinal direction of said subject support. The determined geometric attribute(s) may comprise at least one value indicative of the position and/or of the orientation of the detected object associated therewith. The determined geometric attribute(s) may comprise a centerline of the detected object associated therewith.

For example, the processor 12 may be adapted for detecting the object(s) by determining 3D points for which the corresponding acquired depth information differs substantially from the corresponding reference depth information. Then, it may associate the identified points, and/or clusters of said points (e.g. determined by a clustering algorithm), with the object(s) being detected.

The processor may be adapted to repeat the steps of acquiring the image and/or spatial data, via the camera system 11, and detecting the object(s), in which, after each iteration, the reference data is updated to include the detected object(s), such that the updated reference data represents the subject support with the object(s) detected up to that iteration, and such that, in a next iteration, one or more further objects can be placed on the subject support to be detected.

The device may comprise a user interface, e.g. which may comprise a display monitor, a mouse, a keyboard and/or one or more similar human interface devices known in the art. The user interface may be adapted to control the processes discussed hereinabove and below, e.g. to configure and/or control the detection of object(s) and the attributes determined therefor. The user interface may be adapted for interacting with an operator, e.g. a healthcare professional, such as a medical doctor, a nurse, a radiotherapist, an imaging technologist or the like. For example, the user interface may be used by the operator to control the device and/or monitor the positioning of the patient and equipment items in the preparation phase of the imaging/therapy session.

The output 13 may, for example, comprise a display monitor 15. The processor 12 may be adapted for presenting, via the display monitor, a visual representation of the determined at least one geometric attribute for the detected object or objects to an operator. The processor 12 may be adapted for repeatedly performing the image and/or spatial data acquisition, determining the at least one geometric attribute, and presenting the at least one geometric attribute, so as to dynamically guide the operator in positioning and/or orienting the detected object and/or objects.

For example, the shape, area and/or volume of the detected object(s) may be presented, i.e. visualized, as a color overlay or contour on the at least one camera image and/or model of the scene. The processor 12 may be adapted for presenting the visual representation in an overlay on at least one camera image as acquired by the camera system and/or on a geometric model abstracted from the acquired image and/or spatial data and/or from the obtained reference data. For example, the at least one geometric attribute may comprise a centerline of the (or each) detected object, which may be represented by a straight, segmented or curved line, or a set of points indicative of such line. Other examples include a center point of the object indicated at its corresponding location in the image or model, an orientation vector, and/or another suitable marking indicating the determined attribute.

The device may also comprise a data storage interface 16. The processor may be adapted for storing the at least one geometric attribute in a data storage via the data storage interface, for future reference. For example, the device may comprise the data storage, i.e. the data storage may be integrated in the device, or may comprise an interface for accessing an external data storage, e.g. via a data communication network. For example, the data storage unit may comprise a data storage disk, a hard drive, a database, a network drive or any other suitable means for storing digital information.

For example, the processor 12 may be adapted to retrieve the at least one geometric attribute stored in a previous (e.g. imaging and/or therapy) session, and to include this retrieved information in the visual representation for comparative purposes.

In a third aspect, the present invention relates to a medical imaging and/or radiotherapy system, in which the system is adapted to perform a method in accordance with embodiments and/or comprises a device in accordance with embodiments of the present invention. In a fourth aspect, the present invention relates to a workstation for a medical imaging and/or radiotherapy system, in which the workstation is adapted to perform a method in accordance with embodiments of the first aspect of the present invention and/or comprises a device in accordance with embodiments of the second aspect of the present invention.

Thus, embodiments of the present invention also relate to a magnetic resonance imaging workstation or magnetic resonance imaging system, a computed tomography workstation or computed tomography system, a radiotherapy workstation or radiotherapy system, and/or similar imaging and/or thereapy workstation or system, in which the workstation or system comprises a device in accordance with embodiments of the present invention.

In a fifth aspect, the present invention relates to a computer-program product for performing, when executed on a computer (e.g. a device in accordance with embodiments of the second aspect of the present invention), a method in accordance with the first aspect of the present invention.

Other features, or details of features, described hereinabove of a device (resp. computer-program product, system and workstation) in accordance with embodiments of the present invention shall be clear in view of the description provided hereinabove relating to a method in accordance with embodiments of the present invention, and/or vice versa.

As an example to illustrate embodiments of the present invention, Fig. 3 shows a procedure in which a knee positioning device is used in an imaging or therapy session, e.g. an MRI or a radiotherapy session. In accordance with an embodiment of the present invention, image information is gathered, e.g. visible spectrum image data and corresponding depth imaging data (or a setup is used in which such information is implicitly gathered in conjunction, e.g. using stereo imaging). For example, the session preparation may be performed by first placing the knee device in a correct position on the table, followed by, in a next step, positioning the patient on the prepared patient support. A method as discussed hereinabove may be applied to detect the knee positioning device, e.g. by detecting a point cloud that is elevated with respect to the table, and to determine relevant geometric attribute(s) thereof.

Different possible positions in the longitudinal direction are indicated in the different images, e.g. from H3 ("Head 3") to F6 ("Feet 6"). The detected shape 202 of the device may be filled by a (e.g. transparent) color in a view as presented to the operator. For example, the (e.g. live) camera image may be presented in monochrome (e.g. greyscale), whereas the detected object 202 (e.g. the segmented shape or volume of the knee support) may be shown in a color overlay, e.g. purple.

The center of mass as determined by the method 100, may be indicated by a marker 201, e.g. a colored dot, e.g. in a color different from the color used to show the detected shape 202, e.g. yellow. The position indicator (H3 - F6, e.g. the discrete subject support index discussed hereinabove) may also be computed, e.g. as the nearest position in this discrete set of possible positions to the detected center of mass. Thus, position indicator may also be displayed and/or used for other purposes, e.g. stored in an annotation.

When a subject is positioned on the prepared patient support, the point cloud corresponding to the subject may be likewise detected. Then, geometric attributes of the subject may be computed from the detected point cloud. The computation of a centerline of the point cloud may be particularly useful as such geometric information, in order to facilitate correct alignment of the subject. This may be generally used for objects that have a substantial flexibility or can be non-rigidly deformed, e.g. the patient (but not necessarily limited thereto).

In Fig. 4, the positioning of a patient on the patient support is assisted by the method in accordance with embodiments. In the situation shown on the left, the centerline of the table and the centerline of the patient substantially overlap. The detected shape 202 of the patient (e.g. as determined by segmentation or any other suitable detection algorithm) may be shown in a (e.g. transparent) color overlay (e.g. purple) on a camera image, in a manner similar to the example of the knee support in Fig. 3. Alternatives may also be easily envisioned, e.g. showing a contour of the shape on the image.

The geometric attribute(s) determined from the detected body (2D or 3D) shape may include (a) value(s) representative of a centerline of the body. This approach, e.g. detection and/or display of centerlines, may be applied to each detected object of interest in a case where multiple objects are detected, however, in other embodiments of the present invention, the centerline detection and/or presentation thereof (e.g. display on a camera image), may be specifically applied to only the patient's body and not to other objects, e.g. to positioning aids, which may be described by other geometric attributes, e.g. position coordinates, an orientation vector, one or more size indicators and/or other quantities indicative of the spatial properties of an object, e.g. applicable to any generic physical object.

Since the patient's body can be easily discerned from other detected objects, a differential treatment in processing and/or presentation of information relating to the patient and information relating to auxiliary devices, e.g. positioning devices, may be easily applied, cf. some of the possible approaches discussed hereinabove (without limitation thereto). It will be understood that, instead of a differential detection of the patient and other, inanimate objects, the method may also rely on an input of the operator to identify the type of object (e.g. a selector to indicate that the patient or another object is being placed in the scene), may be configured to expect a sequence of objects (incl. the patient) in a predetermined order, or may avoid the need for differential detection altogether by treating all detected (or detectable) objects equally.

Fig. 4 shows a centerline of the body, e.g. based on the corresponding segmented (2D or 3D) shape, in the form of dots 207 along a (generally curved and/or segmented) line. The centerline is, at least in this example, considered to be the centerline of the body in the longitudinal direction, e.g. the inferior-superior body axis and/or the centerline with respect to the longitudinal table axis, or is a suitable approximation thereof or proxy therefor. For example, a simple approach to determine such centerline dots is to consider a plurality of lines (for 2D imaging based methods) or planes (e.g. for 3D-based approaches) orthogonal to the centerline of the patient table, for example equidistantly spaced lines or planes, and determine, for each such line or plane, the center point of the intersection (being a line segment or cross-sectional area) between the line or plane and the detected body (e.g. the body component of a segmentation map or other suitable representation of the detected object). Thus, these center points, which may be calculated as the geometric center (i.e. centroid) of the line segment or cross-sectional area, may form the dots 207 for the centerline representation. Alternatives can be easily found to determine and/or present the body centerline more accurately and/or in a manner that is less dependent on the table orientation. For example, optimization or other suitable techniques may be used to find a curved and/or segmented line (e.g. a piecewise continuous line) that maximizes symmetry (e.g. mirror or planar point symmetry, e.g. left-right mirror symmetry) of the detected body with respect to that line. However, it can be appreciated that an approach, for example as discussed hereinabove, that is based on center point sampling along predetermined planes/lines, e.g. as easily determined from the (known) longitudinal table axis, may be particularly simple and efficient, and may provide sufficient accuracy for the intended purposes in many use cases.

As another example, let the main longitudinal axis of the patient support be denoted by X. This may typically correspond, at least approximatively, to the feet-head (inferior-superior) direction of the patient. First, X-coordinates of the extracted points (e.g. a point cloud cluster) corresponding to the subject may be computed. Then, a set of discrete X_{b} positions, ranging from the minimum to the maximum X-coordinate of the patient support or of the detected points in the point cloud representing the body, may be defined to define a number of bins. Each 3D point of the point cloud representing the patient may thus be assigned to a bin, based on its X-coordinate. For each bin, the average Y- and/or Z-coordinates, denoted *Y̅_{b}* and *Z̅_{b}* of the points associated with this bin may be computed to represent the center line. Particularly, the center line of the subject may be computed as the set of points (*X_{b}, Y̅_{b}, Z̅_̅{̅b̅}̅*), e.g. one such point per bin, and can be shown on an (e.g. camera) image guiding the operator in the process of accurate alignment of the patient on the subject support.

An ideal center line, e.g. corresponding to the centerline 205 of the patient support, can be displayed as well, e.g. for visual reference. The table centerline 205 may be static, e.g. unchanging between different procedures using the same system and/or in the same imaging/therapy environment, or may relate in a predetermined manner to system parameters that can be obtained via a suitable interface, e.g. taking a received position of an automated couch actuation system into account. Thus, the centerline 205 of the table may be preconfigured, e.g. defined in configuration data and/or as an element in the reference data describing the "empty" table, e.g. reference data obtained by a calibration procedure. This may be a static definition, or a definition of parameters that define a relation to obtainable system parameters, e.g. defining a position and orientation of the line to be displayed in/on the image as a function of table height and/or lateral and/or longitudinal coordinates (of an automated table actuation system), without limitation to this example. It will be understood that this table centerline may also be determined as part of the segmentation and/or other camera image/data processing, even though the aforementioned predetermined configuration of the centerline and/or a predetermined relation to readily available system parameters may typically suffice.

For example, by determining the centerline of the patient (e.g. visualized as dots 207), the alignment of the patient can be easily checked. In the left-hand photograph shown in Fig. 4, an alignment of the patient's body along substantially a straight line is shown, in which the centerline also (approximately) coincides with the table axis. This may be, in at least some scenarios, a preferred alignment of the patient for the imaging or therapy procedure. Any substantial deviation from this straight line and a proper lateral centering of the body can thus be easily detected by the operator.

However, the photograph on the right side in Fig. 4 shows significant deviations from the target centerline position. The centerline representation 207 of the body is clearly not straight, and not along the table centerline. Some potential reasons for this can easily be seen in the visual presentation: the patient's head may be slightly tilted toward the left side (the upward page direction), and the overall orientation of the upper body appears to be biased to the left table side as well (i.e. an increasing displacement to the left toward the head of the patient), instead of straight and aligned with the longitudinal axis. In many situations such problems would be less obvious without the aid of such visualization, even with a raw overhead observation camera feed to give the operator a birds-eye perspective. However, in accordance with embodiments of the present invention, the operator can easily see that the centerlines of the subject and the table do not overlap, that a corrective action could be to slightly rotate the upper body to the right side, and draws attention to a potentially slightly sideways tilted head. Similarly, it can be easily seen that the right leg is not fully extended and the feet are not symmetrically positioned and oriented.

Whereas only the XY (horizontal) plane is shown in Fig. 4, it will be understood that the depth, i.e. vertical (Z), component, e.g. of the detected center line of the body, can be visualized as well, thanks to the use of depth data. Therefore, it is an advantage that embodiments of the present invention enable a positioning of the patient (and/or any relevant devices) to be guided and/or verified in a convenient manner in the horizontal plane as well as in the vertical direction. For example, the Z-component of the detected center line may be indicated on a similar XZ image (synthetic or obtained by a camera at a different, suitable, vantage point), as a simple graph of Z as function of X, and/or in another suitable form. For example, the dots representing the center line may also be annotated with height information, shown in a color as dependent on the corresponding Z-value, or varied in size as function of Z, among other possibilities.

## Claims

1. A method (100) for assisting in the positioning and/or orienting of a plurality of objects, including a subject and/or at least one auxiliary equipment item, on a subject support in an imaging and/or therapy session, the method comprising:
- acquiring (101) image and/or spatial data, using a camera system, of the subject support, having at least one of said objects placed thereon, wherein said image and/or spatial data comprises depth and/or three-dimensional, 3D, information,
- obtaining (111) reference data representative of the subject support without said at least one object placed thereon, the reference data comprising reference depth and/or 3D information,
- determining (104), from the image and/or spatial data, at least one geometric attribute of the at least one object,
wherein said determining (104) of the at least one geometric attribute comprises detecting (105) said at least one object by at least taking the reference depth and/or 3D information in said reference data and the depth and/or 3D information in said acquired image and/or spatial data into account.

2. The method of claim 1, wherein said detecting (105) comprises determining (110) three-dimensional points for which the corresponding acquired (101) depth and/or 3D information differs from the corresponding reference depth and/or 3D information and associating said points, and/or determined clusters of said points, with said at least one object.

3. The method of any of the previous claims, wherein the steps of acquiring (101) the image and/or spatial data and detecting (105) at least one of the objects are repeated (114), in which, after each iteration, said reference data is updated (113) to include the detected object or objects, such that the updated reference data represents the subject support with thereon the object or objects detected up to said iteration, and such that, in a next iteration, one or more further objects can be placed on the subject support to be detected (105).

4. The method of any of the previous claims, wherein said at least one geometric attribute comprises:
- a discrete subject support index, selected from a predetermined discrete ordinal set of subject support indices, to identify a position of the detected object associated with said at least one geometric attribute along a longitudinal direction of said subject support, and/or
- at least one value indicative of the position and/or of the orientation of the detected object associated therewith, and/or
- a centerline of the detected object associated therewith.

5. The method of any of the previous claims, comprising presenting (107) the determined (104) at least one geometric attribute for the detected (105) object or objects to an operator, wherein the image and/or spatial data acquisition (101), determining (104) the at least one geometric attribute, and presenting (107) the at least one geometric attribute are repeatedly (106) performed, so as to dynamically guide the operator in positioning and/or orienting the detected object and/or objects.

6. The method of claim 5, comprising storing (108) the at least one geometric attribute in a data storage and/or comprising retrieving (109) said at least one geometric attribute stored (108) in a previous session for presenting said retrieved information in said presentation (107) for comparative purposes.

7. A device (10) for assisting in the positioning and/or orienting of a plurality of objects, including a subject and/or at least one auxiliary equipment item, on a subject support in an imaging and/or therapy session, the device comprising:
- a camera system (11) for acquiring image and/or spatial data of the subject support, having at least one of said objects placed thereon, wherein said camera system is adapted for acquiring depth and/or three-dimensional, 3D, information,
- a processor (12), and
- an output (13),
wherein said processor is adapted for obtaining reference data representative of the subject support without said at least one object placed thereon, the reference data comprising reference depth and/or 3D information,
wherein said processor is adapted for detecting said at least one object by at least taking the reference depth and/or 3D information in said reference data and the depth and/or 3D information in said acquired image and/or spatial data into account, for determining at least one geometric attribute of the at least one object, and for outputting, via the output (13), said at least one geometric attribute.

8. The device of claim 7, wherein said processor (12) is adapted for detecting said at least one object by determining three-dimensional points for which the corresponding acquired depth and/or 3D information differs substantially from the corresponding reference depth and/or 3D information, and associating said points and/or determined clusters of said points with said at least one of said objects being detected.

9. The device of claim 7 or claim 8, wherein said processor (12) is adapted to determine said at least one geometric attribute, the at least one geometric attribute comprising:
- a discrete subject support index, selected from a predetermined discrete ordinal set of subject support indices, to identify a position of the detected object associated with said at least one geometric attribute along a longitudinal direction of said subject support, and/or
- at least one value indicative of the position and/or of the orientation of the detected object associated therewith, and/or
- a centerline of the detected object associated therewith.

10. The device of any of the claims 7 to 9, wherein said camera system (11) comprises at least one camera arranged above the subject support such as to obtain a two-dimensional and/or three-dimensional overhead view of the subject support.

11. The device of any of the claims 7 to 10, comprising an input (14) for receiving a current position of the subject support from an automated subject support actuation system and/or wherein said processor is adapted for determining said current position from said acquired image and/or spatial data,
wherein said processor (12) is adapted for determining said reference depth and/or 3D information based on said current position and a three-dimensional geometric model of the subject support and/or based on said current position and a set of reference depth images of the subject support corresponding to a plurality of different positions.

12. The device of any of the claims 7 to 11, wherein said output (13) comprises a display monitor (14), and wherein said processor (12) is adapted for presenting, via said display monitor, a visual representation of the determined at least one geometric attribute for the detected object or objects to an operator, wherein said processor is adapted for repeatedly performing the image and/or spatial data acquisition, determining the at least one geometric attribute, and presenting the at least one geometric attribute, so as to dynamically guide the operator in positioning and/or orienting the detected object and/or objects.

13. The device of any of the claims 7 to 12, comprising a data storage interface (15), wherein said processor (12) is adapted for storing the at least one geometric attribute in a data storage, via said data storage interface (15) for future reference.

14. A medical imaging and/or radiotherapy system, or a workstation for such system, wherein said system or workstation is adapted to perform a method in accordance with any of the claims 1 to 6.

15. A computer-program product for performing, when executed on a computer, the method of any of the claims 1 to 6.
